# EUROPEAN PATENT APPLICATION

(11) **EP 3 428 189 A1**
(43) Date of publication of application: **16.01.2019**
(21) Application number: 18190279.2
(22) Date of filing: 28.10.2015
(51) Int. Cl.: C07K 16/24, A61K 39/395, A61K 47/10, A61K 47/18, A61K 47/00, A61K 39/00

(54) **PHARMACEUTICAL ANTI-TNF-ALPHA ANTIBODY FORMULATION**

(30) Priority: 28.10.2014 HU 1400510
(62) Divisional of application: 15808356.8
(71) Applicant: Richter Gedeon Nyrt., 1103 Budapest (HU)
(72) Inventor: LÁZÁR, József, 1036 Budapest (HU); OLAJOS, Marcell, 1181 Budapest (HU); JÁNOS, Varga-Kugler, 1202 Budapest (HU)
(74) Representative: Steinecke, Peter

(57) **Abstract**

Provided are improved storage-stable liquid pharmaceutical antibody formulations. In particular, liquid aqueous pharmaceutical formulations of Adalimumab based on alternative buffer systems to citrate/phosphate and pharmaceutical containers like auto-injection devices containing the same are described.

## Description

### Field of the Invention

The present invention relates generally to the field of pharmaceutical formulations. Specifically, the present invention relates to improved storage-stable liquid pharmaceutical antibody formulations. This invention is exemplified by stabilized liquid pharmaceutical formulations of an anti-TNFa antibody. In particular, the current invention discloses liquid aqueous pharmaceutical formulations of Adalimumab based on alternative buffer systems to citrate/phosphate and pharmaceutical containers like auto-injection devices containing the same.

Several documents are cited throughout the text of this specification. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

### Background of the Invention

Many proteins in solution are marginally stable, undergoing conformational changes due to various stresses during purification, processing and storage. Such stresses can include elevated temperature, shear strain, surface adsorption, and high protein concentration (Arakawa T et al. 2006). Structurally altered proteins have a strong tendency to aggregate, often leading to eventual precipitation. Irreversible aggregation is a major problem for long-term storage stability of therapeutic proteins and for their shipping and handling.

As highly complex proteins, antibodies are susceptible to a variety of physical and chemical degradation pathways. Antibody aggregation easily occurs during storage in a liquid state (Chen B et al. 2003), leading to a loss of biological activity and increasing immunogenicity which in turn can cause serious adverse reactions like anaphylactic shock and other safety issues. Therefore there is a need to develop pharmaceutical antibody formulations which reduce antibody aggregation. The major aim of formulating antibodies as therapeutic agents is to maintain antibody solubility, stability and bioactivity, particularly at high antibody concentrations, and control the rate of antibody degradation to provide an acceptable shelf life for worldwide shipping and storage.

To prevent the formation of aggregates that can cause undesirable immunogenicity or altered half-life, pharmaceutical antibody formulations usually contain one or more buffer(s) to maintain a given pH range and an isotonizing agent to provide a similar osmolarity as physiological liquids. A variety of formulation excipients have been shown to stabilize antibodies under different processing conditions and during storage, including sugars, polyols, amino acids, and surfactants (Paborji M et al. 1994; Sarno MEC et al. 1999). Since aggregation highly depends on antibody concentration and the pH of the formulation, the selection of a suitable buffer system is a crucial step towards a stable formulation, especially with regard to highly concentrated monoclonal antibody formulations suitable for subcutaneous (sc) administration like in the case of the anti-TNFα antibody Adalimumab (HUMIRA®, AbbVie Inc; 50mg/mL), which is formulated in a citrate and phosphate buffer (WO2004/016286). Disadvantages of buffer systems comprising citrate and/or phosphate are well known in the art with a view to injection cite pain induced by citrate buffer (Kappelgaard A 2004, Frenken et al. 1993) and phosphate buffer (Fransson et al. 1996) and pH instability of phosphate-based buffers upon freezing (Kolhe P et al. 2010). The stability and safety of the pharmaceutical drug product thus depends on both type and concentration of a buffering agent.

As for chronic diseases such as rheumatoid arthritis, psoriasis, or ulcerative colitis where medication eventually has to be administered life-long and where home (self) medication is desirable with respect to patient compliance and cost reduction, subcutaneous administration is the preferred route of administration. Due to tissue backpressure and injection pain however, drug administration via the sc route is limited by an injection volume of 1 ml up to 1.5 ml (Gatlin LA and Gatlin CAB 1999). Monoclonal antibody products approved for the treatment of rheumatoid arthritis, psoriasis, or ulcerative colitis like HUMIRA® require relatively high clinical doses (5-700 mg per patient, administered either as fixed dose or on the basis of body weight (kg) or body surface (m²), (Roskos LK et al. 2004).

To address these requirements with particular regard to the suitability for (life-long self-administration) and for the full exploitation of the clinical potency of an antibody like Adalimumab, there is a need for the development of liquid aqueous pharmaceutical formulations of high concentrated antibodies with improved storage stability. The present invention addresses the above-identified need by providing new high concentrated pharmaceutical formulations of Adalimumab with increased stability within suitable pharmaceutical containers (auto-injector devices) which overcome the disadvantages of formulations known from the prior art.

### Summary of the Invention

The present invention discloses a stable liquid aqueous pharmaceutical formulation which is free or substantially free of a phosphate buffer and comprises a therapeutically effective amount of Adalimumab in a buffered solution comprising L-Histidine and (i) citrate and/or (ii) acetate, wherein the stable liquid aqueous pharmaceutical formulation is having a pH of 5 to 5.5.

In a further preferred embodiment of the present invention, said Adalimumab pharmaceutical formulation shows an aggregate content upon storage at about 5 °C for at least 6 months of about less than 5% +/- 0.5% compared to the aggregate content measured at the start of the experiments.

In a further preferred embodiment of the present invention, said Adalimumab pharmaceutical formulation shows an aggregate content upon storage at about 25 °C for at least 6 months of about less than 5% +/- 0.5% compared to the aggregate content measured at the start of the experiments.

In a further preferred embodiment of the present invention, said Adalimumab pharmaceutical formulation shows a content of acidic species upon storage at about 5 °C for at least 6 months of about less than 40% +/- 0.5% compared to the content of acidic species measured at the start of the experiments.

In a further preferred embodiment of the present invention, said Adalimumab pharmaceutical formulation shows a content of acidic species upon storage at about 25 °C for at least 6 months of about less than 40% +/- 0.5% compared to the content of acidic species measured at the start of the experiments.

In a further preferred embodiment of the present invention, said Adalimumab pharmaceutical formulation has enhanced stability of at least 3 to 6 months at a temperature of about 5 °C or 25°C. Basis of the present invention is the observation that liquid aqueous pharmaceutical compositions of Adalimumab which are free or substantially free of a phosphate buffer and which are formulated in L-Histidine-based buffer systems show increased long-term stability in terms of aggregate formation and generation of acidic species.

As shown by HP-SEC analysis and demonstrated within the Tables referred to in the Examples and Figures, the phosphate-buffer free pharmaceutical formulations of Adalimumab as disclosed by the present invention are advantageous over the citrate-phosphate buffer based pharmaceutical Adalimumab reference formulation composed according to the formulation of marketed product HUMIRA®. In particular, the pharmaceutical Adalimumab formulations of the present invention which were buffered with Histidine-citrate or Histidine-acetate buffer were more stable as the reference formulation of Adalimumab buffered with citrate and phosphate (#01).

In one embodiment, the pharmaceutical formulation of Adalimumab of the present invention which was buffered with Histidine-citrate (#07) showed about 5% to 10% lower amount of aggregates upon storage for 1, 2, 3, 6 months at 5°C and about 10% to 20% upon corresponding storage at 25°C than the citrate-phosphate buffered reference pharmaceutical formulation; see Tables 10 to 13 in Example 5.

In another embodiment, the pharmaceutical formulation of Adalimumab of the present invention which was buffered with Histidine-acetate (#10) showed about 5% to 10%; lower amount of aggregates upon storage for 1, 2, 3, 6 months at 5°C and about 10% to 20% upon corresponding storage at 25°C than the citrate-phosphate buffered reference pharmaceutical formulation; see Tables 10 to 13 in Example 5.

In one embodiment, the pharmaceutical formulation of Adalimumab of the present invention which was buffered with Histidine-citrate (#07) showed about 2% to 5% (ca. 4%) lower amount of acidic species upon storage for 3 and 6 months, respectively, at 25°C than the citrate-phosphate buffered reference pharmaceutical formulation; see Table 16 in Example 7.

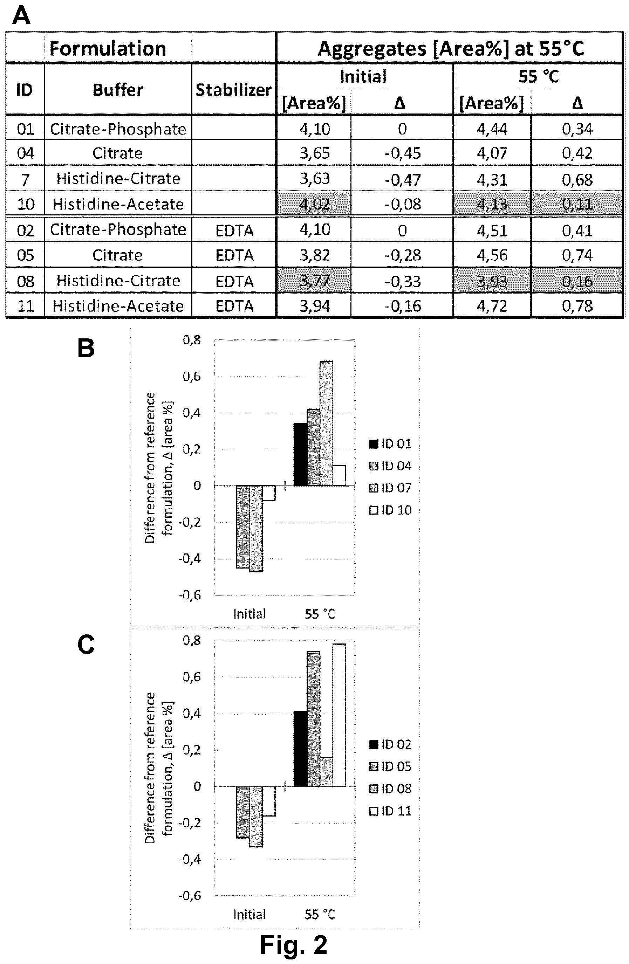

According another embodiment, the pharmaceutical formulation of Adalimumab of the present invention which was buffered with Histidine-acetate (#10) showed about 4% to 8% (ca. 7%) lower amount of acidic species upon storage for 3 and 6 months, respectively, at 25°C than the citrate-phosphate buffered reference pharmaceutical formulation; see Table 16 in Example 7. In one embodiment, the pharmaceutical formulation of Adalimumab of the present invention which was buffered with Histidine-citrate (#07) showed about 10% to 15% (ca. 12,5%) lower aggregate content under heat stress conditions (55°C) when compared to the citrate-phosphate buffered reference pharmaceutical formulation; see Example 6 and the Table in Fig. 2A.

In a further embodiment, the pharmaceutical formulation of Adalimumab of the present invention which was buffered with Histidine-acetate (#10) showed about 4% to 8% (ca. 7%) lower aggregate content under heat stress conditions (55°C) when compared to the citrate-phosphate buffered reference pharmaceutical formulation; see Example 6 and the Table in Fig. 2A.

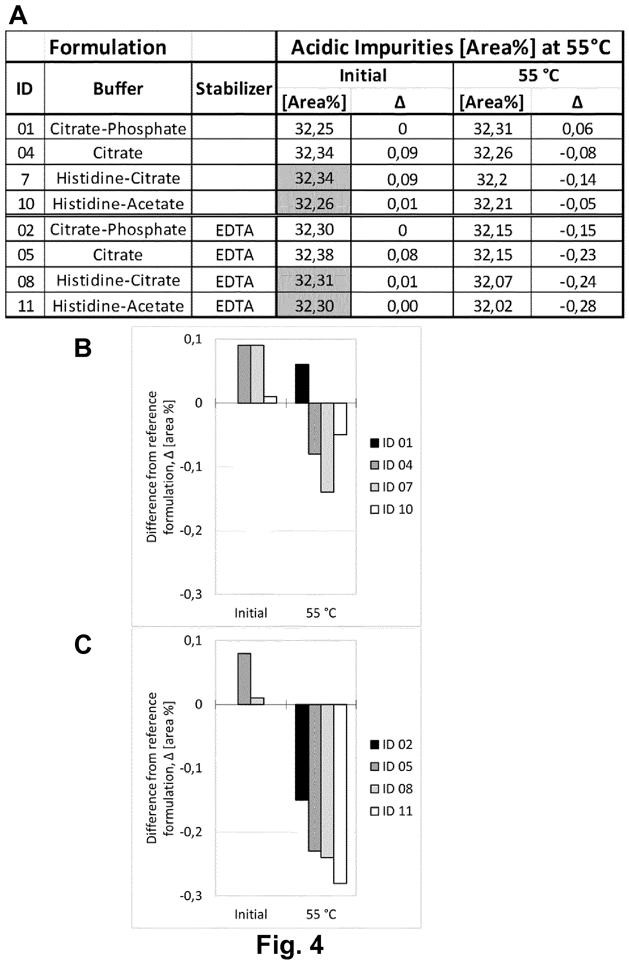

According to another embodiment, the pharmaceutical formulations of Adalimumab of the present inventions which were buffered with Histidine-citrate (#07) or Histidine-acetate (#10) showed a lower amount of acidic species under heat stress at 55°C than the citrate-phosphate buffered reference pharmaceutical formulation; see Example 8 and the Table in Fig. 4A.

In addition, in one embodiment of the present invention, the alternative Adalimumab pharmaceutical formulation has at least one feature selected from the group consisting of: improved long-term stability, better (heat stress) temperature stability, and/or decreased formation of aggregates, and/or decreased formation of acidic species compared to a reference formulation comprising a citrate-phosphate buffer system, preferably as indicated in the embodiments above and illustrated in the Examples and Figures, respectively.

In addition, the stable liquid aqueous pharmaceutical formulation comprising Adalimumab of the present invention which is free or substantially free of a phosphate buffer comprises further a polyol, a surfactant and sodium chloride.

In another embodiment, the stable liquid aqueous pharmaceutical formulation comprising Adalimumab of the present invention which is free or substantially free of a phosphate buffer comprises further EDTA or L-Arginine.

In various embodiments of the present invention stable liquid (aqueous) pharmaceutical formulations can be prepared comprising 1-150 mg/mL of Adalimumab and having a Histidine-citrate or Histidine-acetate buffer with a desired pH (e.g., within the range of pH 5 to pH 5.5), and further comprising a polyol (mannitol 5-20 mg/mL), a surfactant (Polysorbate 80 0.1-10 mg/mL), sodium chloride (5-7.5 mg/mL), with or without EDTA (1-50 mg/mL, preferably 10 mg/mL) or L-Arginine (0.5-6.5 mg/mL).

In a further aspect of the present invention, the pharmaceutical Adalimumab formulation is designed for parenteral administration, preferably for intravenous injection, or for intramuscular injection, more preferably for subcutaneous administration for use in the treatment of a disease selected from the group consisting of autoimmune disorders and malignant diseases.

Another aspect of the present invention provides a prefilled syringe or autoinjector device, containing any of the stable pharmaceutical Adalimumab formulations disclosed within the present invention.

In certain embodiments of the present invention, the liquid aqueous pharmaceutical formulation comprising Adalimumab is contained in a prefilled pharmaceutical container.

In one embodiment of the present invention, the prefilled pharmaceutical container is a prefilled syringe, injection pen, ampoule, bottle, autoinjector or an infusion bag which comprises the liquid aqueous pharmaceutical formulation according to the first aspect of the invention at concentrations about 20 mg, or about 40 mg or about 80 mg Adalimumab.

For example, the invention provides an autoinjector device comprising a liquid aqueous pharmaceutical formulation comprising Adalimumab at a concentration of between about 1 and about 150 mg/mL, a Histidine-citrate buffer or a Histidine-acetate buffer, sodium chloride, mannitol and Polysorbate 80, wherein the pH of the pharmaceutical formulation is about pH 5.2.

In detail, the present invention is directed to:
[1] A pharmaceutical formulation which is free or substantially free of a phosphate buffer and selected from the group consisting of:
   (a) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of aggregated species of Adalimumab after storage at 5°C for 3 to 6 months of about less than 5 %;
   (b) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of aggregated species of Adalimumab after storage at 25°C for 3 to 6 months of about less than 5%;
   (c) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of acidic species of Adalimumab after storage at 25°C for 3 to 6 months of about less than 40%, preferably less than 35%;
   (d) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of aggregated species of Adalimumab under heat stress conditions at 55°C of about less than 5%;
   (e) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of acidic species of Adalimumab under heat stress conditions at 55°C of about less than 40%, preferably less than 35%;
   (f) a liquid aqueous pharmaceutical formulation comprising a therapeutically effective amount of Adalimumab in a buffer solution comprising L-Histidine and (i) citrate and/or (ii) acetate, said formulation having a pH of 5 to 5.5 and having enhanced stability of at least 6 months at a temperature of about 5°C or 25°C; and
   (g) a liquid aqueous pharmaceutical formulation comprising a therapeutically effective amount of Adalimumab in a buffer solution comprising L-Histidine and (i) citrate and/or (ii) acetate, said formulation having a pH of 5 to 5.5 and retaining its activity of neutralizing the biological activity of TNFα to about 80%, preferably of about 85%, more preferably of about 90%, most preferably of about 95% after storage of 6 months at a temperature of about 5°C and/or 25°C, and/or after being subjected to heat stress at 55°C, freeze-thaw conditions/stress at - 20°C to 5°C and/or mechanical stress.
[2] The formulation of [1], wherein the concentration of the antibody is between about 1-150 mg/mL.
[3] The formulation of [1] or [2], wherein the concentration of the antibody is about 50 mg/mL.
[4] The formulation of any one of [1] to [3], which further comprises a polyol, a surfactant and sodium chloride.
[5] The formulation of [4], wherein the polyol is mannitol and the surfactant is Polysorbate 80.
[6] The formulation of [5], which contains 5-20 mg/mL of mannitol, 0.1-10 mg/mL of Polysorbate 80 and 5 to 7.5 mg/mL sodium chloride.
[7] The formulation of [6], which contains about 12 mg/mL of mannitol, about 1 mg/mL of Polysorbate 80 and about 6.2 mg/mL sodium chloride.
[8] The formulation of any one of [1] to [7], which further comprises (f) L-Arginine.
[9] The formulation of any one of [1] to [8], which further comprises EDTA.
[10] The formulation of any one of [1] to [9], which further is suitable for single use subcutaneous injection and contained in a pharmaceutical container.
[11] A prefilled pharmaceutical container comprising a liquid aqueous pharmaceutical formulation having a pH of 5 to 5.5, wherein the composition is free or substantially free of a phosphate buffer and comprises
   (a) Adalimumab;
   (b) a polyol;
   (c) a surfactant;
   (d) a buffer component comprising L-Histidine and (i) citrate and/or (ii) acetate;
   (e) sodium chloride.
[12] The container of [11], wherein the formulation further comprises (f) L-Arginine.
[13] The container of [11] or [12], wherein the formulation further comprises EDTA.
[14] The container of any one of [11] to [13], wherein the formulation is free or substantially free of dicarboxylic acids, monosaccharides, oligosaccharides and/or additional amino acids.
[15] The container of any one of [11] to [14], wherein the formulation comprises
   (a) 20 to 130 mg/mL of Adalimumab;
   (b) 10-14 mg/mL of a mannitol;
   (c) 0.1-5 mg/mL of Polysorbate 80;
   (d) 0.1-0.5 mg/mL of L-Histidine-HCl and 0.01-0.025 mg/mL of L-Histidine base;
      (i) 0.1-1.5 mg/mL of citric acid monohydrate and 0.25-3.5 mg/mL of trisodium citrate dihydrate and/or
      (ii) 0.1-0.5 mg/mL sodium acetate trihydrate and 0.01-0.05 mg/mL acetic acid; and
   (e) 6.0-6.4 mg/mL sodium chloride.
[16] The container of [15], wherein the formulation further comprises
   (f) 0.5 - 6.5 mg/mL L-Arginine base and/or
   (g) 1-50 mg/mL, preferably 10 mg/mL EDTA.
[17] The container of any one of [11] to [16], wherein the formulation comprises 45 to 55 mg/mL, preferably 50 mg/mL Adalimumab.
[18] The container of any one of [11] to [17], wherein the formulation further is suitable for single use subcutaneous injection.
[19] The container of any one of [10] to [18], which is a prefilled syringe, injection pen, ampoule, bottle, autoinjector or an infusion bag.
[20] The container of any one of [10] to [19], which is closed under nitrogen protective atmosphere.
[21] The container of any one of [10] to [19], wherein the dose strength of Adalimumab is 20, 40, 80 or 160 mg.
[22] A set of at least two containers of any one of [10] to [21], wherein the containers have different dose strengths of Adalimumab.
[23] The set of containers of [22], wherein different dose strengths are constituted by different filling volumes of the same formulation.
[24] The set of containers of [22] or [23], wherein different dose strengths in the prefilled syringes are 20, 40 and/or 80 mg.
[25] The formulation of any one of [1] to [9], the container of any one of claims [10] to [21] or the set of containers of any one of [22] to [24] for use as a medicament for use in the treatment of a disorder in which TNFα activity is detrimental.
[26] The formulation, container or set of containers for use in accordance with [25], wherein the disorder in which TNFα activity is detrimental is selected from the group consisting of an autoimmune or inflammatory disorder.
[27] The formulation, container or set of containers for use in accordance with [25] or [26], wherein the formulation is designed to be administered subcutaneously to the human subject in need thereof on a biweekly dosing regimen of every 13-15 days.
[28] The container or set of containers for use in accordance with any one of [25] to [27] having a dose strength of adalimumab of 20 or 80 mg, and wherein Adalimumab is designed to be administered at a total dose of 20, 40 or 80 mg.
[29] The formulation, container or set of containers for use in accordance with any one of [25] to [28], wherein the formulation is to be administered in combination with an additional therapeutic agent that inhibits TNFα production or activity, preferably methotrexate.

Further embodiments of the present invention will be apparent from the description and Examples that follow.

### Brief description of the drawings

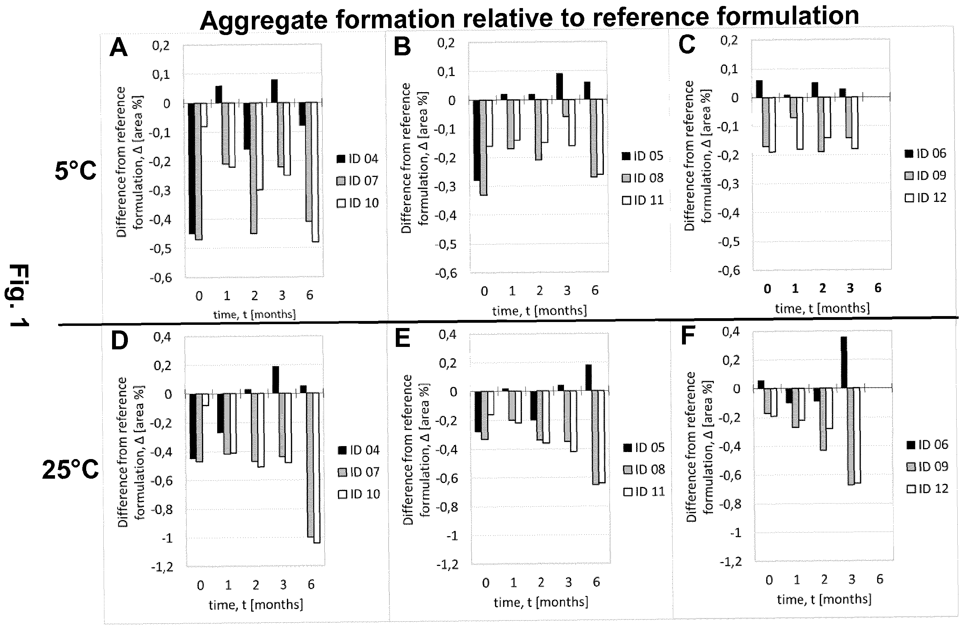

- **Fig. 1:**: Aggregate content of alternative Adalimumab formulations relative to the reference formulation(s) (ID NOs #01- #03, for a detailed summary of buffer components see Tables 11 and 13). Stability of the tested Adalimumab formulations with respect to aggregate formation measured by HP-SEC during storage at 5 °C (A-C) and 25 °C (D-E) over a period of 6 months is shown as relative % areas of aggregated peaks. Sample ID NOs: #01, #04, #07 and #10 represent data of formulations without additives (A, D), sample ID NOs: #02, #05, #08 and #11 represent data of EDTA containing formulations (B, E) and sample ID NOs: #03, #06, #09 and #12 represent data of L-Arginine (L-Arg) containing formulations (C, F; only 3-months stability data available for L-Arg containing formulations). Histidine-citrate (sample ID NO: #07) and histidine-acetate (sample ID NO: #10) buffered Adalimumab formulations have apparently a lower aggregation rate compared to the citrate-phosphate based reference formulations. Of all tested Adalimumab formulations those based on citrate buffer alone are most prone to aggregation. Neither the addition of EDTA (sample ID NOs: #02, #05, #08 and #11) nor of L-Arginine (sample ID NOs:#06, #09, #12; L-Arg as determined after 3 months) has a preventive or stabilizing effect on aggregate formation in the alternative formulations. In conclusion, the histidine-acetate buffered (followed by histidine-citrate buffer) Adalimumab pharmaceutical formulation of the present invention is a more stable alternative to the Adalimumab formulation available on the market.
- **Fig. 2**: Aggregate content of alternative Adalimumab formulations under stress conditions (55 °C) relative to the reference formulation. Aggregate formation upon heating to 55 °C was assessed in formulations without additives (B, sample ID NOs: #01, #04, #07 and #10) and with EDTA (C, sample ID NOs: #02, #05, #08 and #11) using HP-SEC. (A) The aggregate content is shown as area percentages (left columns). The right columns (designated with Δ) represent the differences from the sample in buffer of the reference formulation(s) (#01 and #02) for the initial results and the difference from the initial result (change due to stress) for the stressed samples, respectively. (A, B) Among all tested Adalimumab formulations, the pharmaceutical formulation #10 buffered with histidine-acetate without an additional stabilizer showed the highest stability i.e., the lowest aggregation rate at 55 °C. (A, C) Addition of EDTA to this buffer combination seemingly enhances aggregate formation in the respective formulation while heating to 55 °C (#11). The Adalimumab pharmaceutical formulation buffered with histidine-citrate and comprising EDTA (#08) shows similar stability at 55 °C.

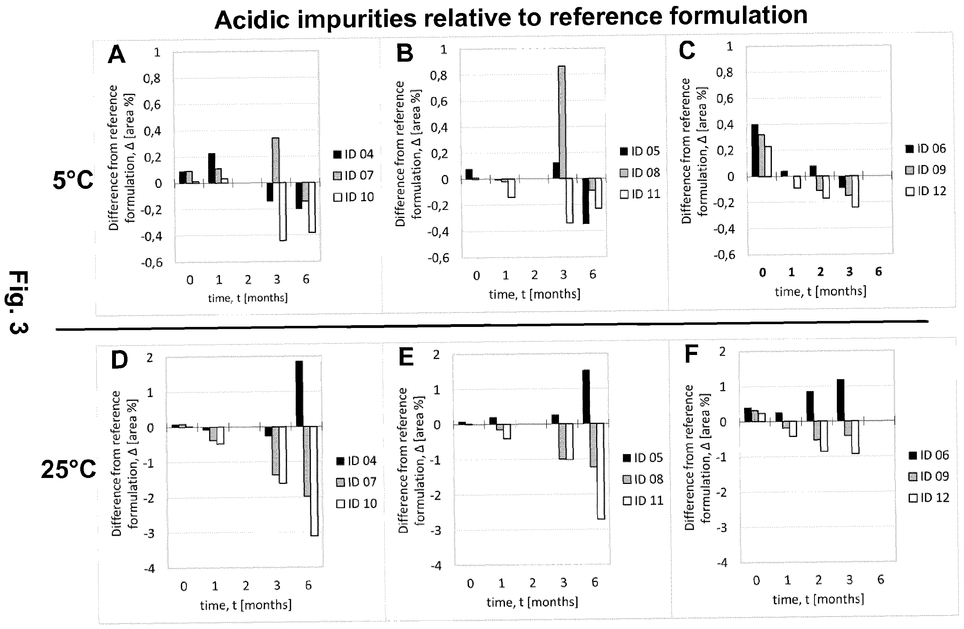

- **Fig. 3**: Acidic impurity/species content of alternative Adalimumab formulations relative to the reference formulation(s) (ID NOs #01-#03), for a detailed summary of buffer components see Tables 15 and 17). Stability of the tested Adalimumab formulations with respect to acidic species formation measured by SCX-HPLC during storage at 5 °C (A-C) and 25 °C (D-F) over a period of 6 months is shown as relative % areas of acidic impurity peaks. Sample ID NOs: #01, #04, #07 and #10 represent data of formulations without additives (A, D), sample ID NOs: #02, #05, #08 and #11 represent data of EDTA containing formulations (B, E) and sample ID NOs: #03, #06, #09 and #12 represent data of L-Arginine (L-Arg) containing formulations (C, F; only 3-months stability data available for L-Arg containing formulations). All tested alternative Adalimumab pharmaceutical formulations stored at 5 °C have apparently slightly lower levels of acidic impurities than the reference formulations comprising citrate-phosphate buffer, however the differences are not significant. Stability studies at 25 °C revealed that histidine-acetate and histidine-citrate based formulations show significantly lower levels of acidic impurities upon storage for 6 months compared to the formulations based on the reference buffer (at least for 3 months in the case of L-Arg containing formulations, respectively). The tested Adalimumab pharmaceutical formulation buffered with Histidine-acetate (#10) has the highest relative purity (3.1 % lower level of acidic impurities/species compared to the reference formulation, whereas Adalimumab formulations based on citrate buffer alone (#04) showed the highest amount of acidic species. Neither the addition of EDTA (sample ID NOs: #02, #05, #08 and #11) nor of L-Arginine (sample ID NOs:#03, #06, #09, #12; L-Arg as determined after 3 months) has a preventive or stabilizing effect on acidic impurity formation in the alternative formulations.
- **Fig. 4**: Acidic species content of alternative Adalimumab formulations under stress conditions (55 °C) relative to the reference formulation. The generation of acidic impurities is the major degradation pathway by deamidation. Acidic impurity content upon heating to 55 °C was assessed in Adalimumab formulations without additives (B, sample ID NOs: #01, #04, #07 and #10) and with EDTA (C, sample ID NOs: #02, #05, #08 and #11) using SCX-HPLC. (A) The acidic impurity content is shown as area percentages (left columns). The right columns (designated with Δ) represent the differences from the sample in buffer of the reference product (#01, #02) for the initial results and the difference from the initial result (change due to stress) for the stressed samples, respectively. (A, B) Among all tested Adalimumab pharmaceutical formulations, the Histidine-Acetate buffered formulation without an additional stabilizer (sample ID NO. #10) showed the highest stability *i.e.* the lowest acidic species formation rate at 55°C. (A-C) All remaining Adalimumab formulations show similar rates of degradation to the investigated stress factor.

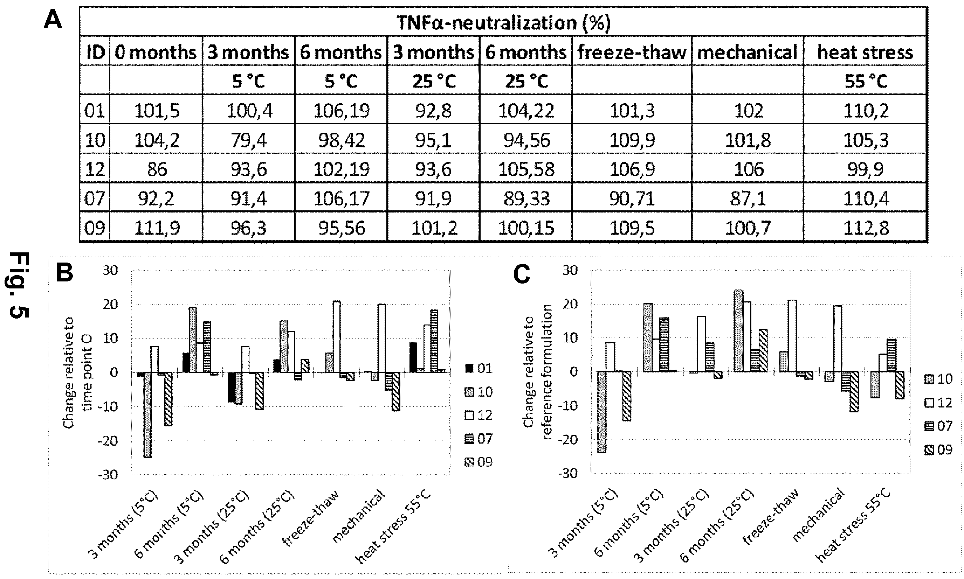

- **Fig. 5**: Biological activity of alternative Adalimumab formulations under stress conditions relative to time and the reference formulation, respectively. The effect of inhibiting cell death by the reference solution and the test sample ID NOs: #01, #07, #09, #10 and #12 was compared on L929 cells treated with TNFα and Actinomycin-D under several different stress conditions, i.e. after 3 months at 5 °C and 25 °C, after 6 months at 5 °C and 25 °C, after a freeze-thaw cycle, after mechanical stress as well as after heat stress at 55°C. (A, B) Relative to time point 0, the Histidine-Acetate buffered formulation without an additional stabilizer (sample ID NO. #10) showed the highest ability to neutralize TNFα after 6 months at 5 °C and 25°C, followed by Histidine-Acetate buffered formulation with L-Arg (sample ID NO. #12). Further, the Histidine-Acetate buffered formulation with L-Arg (sample ID NO. #12) showed the best performance after 3 months at 5 °C and 25 °C, after a freeze-thaw cycle as well as mechanical stress. The Histidine-Citrate buffered formulation without an additional stabilizer (sample ID NO: #07) showed the highest TNFα neutralization after 6 months at 5 °C as well as after heat stress at 55°C. All remaining Adalimumab formulations show similar rates of degradation to the investigated stress factor. (A, C) Relative to the reference formulation, the Histidine-Acetate buffered formulation without an additional stabilizer (sample ID NO. #10) showed the highest ability to neutralize TNFα after 6 months at 5 °C and 25 °C, followed by Histidine-Citrate (sample ID NO. #07) after 6 months at 5 °C. The Histidine-Acetate buffer formulation with L-Arg (sample ID NO. #12) showed overall the best performance to all investigated stress factors. The experiments summarized in Fig. 5 were performed with a second batch of alternative Adalimumab formulations.

### Detailed Description of the Invention

The present invention generally relates to long-term stable pharmaceutical formulations of an anti-TNFa antibody, in particular Adalimumab based on histidine buffer systems. More particularly, in accordance with the present invention phosphate-buffer free pharmaceutical formulations of Adalimumab are provided, which are buffered with Histidine-citrate or Histidine-acetate buffer and show improved stability upon storage at 25°C over a period of at least three to six months compared to a citrate-phosphate buffer based reference pharmaceutical Adalimumab formulation composed according to the formulation of marketed product HUMIRA®.

The present invention is based on the observation that liquid aqueous pharmaceutical formulations of Adalimumab which are free or substantially free of a phosphate buffer and which are formulated in L-Histidine-based buffer systems provides suitable alternative pharmaceutical Adalimumab formulations with increased long-term stability in terms of aggregate formation and generation of acidic species; see Examples 5 to 9 and Figures 1 to 5.

Previously, alternative buffer systems for formulations of Adalimumab *inter alia* comprising histidine either alone or in combination with other buffer components such as acetate, citrate and phosphate have been suggested; see Bender "Alternative buffers for pharmaceutical anti-TNFalpha monoclonal antibody formulations" (www.technik2day.net, published on 06. February 2013, publication number PAPDEOTT002384). However, no data or indication were given on the stability of any histidine buffered formulation of Adalimumab, which could form the starting point for the development of long-term stable pharmaceutical formulations of Adalimumab, *i.e.* a concrete formulation from which more generic histidine buffered formulations could be extrapolated.

Indeed, *hitherto* combined aqueous buffer solutions including histidine and for example acetate or citrate have been discarded as not suitable or being inferior compared to histidine as the sole buffer component or in combination with others. For example, in international application WO2014/039903 at page 88 and 89 in Table H and HI among various others two formulations, formulation 6 and 10 of a proprietary Adalimumab biosimilar with histidine-acetate and histidine-citrate are described in a short-term stability test for only one and two weeks and disregarded for further investigation of a pharmaceutical formulation. Rather, WO2014/039903 teaches the avoidance of for example citrate but to use histidine only or in combination with a succinate buffer. Nevertheless, in one embodiment the pharmaceutical formulation of the present invention does not consist of a formulation according to formulation 6 or 10 in Table H and HI at page 88 and 89 WO2014/039903. Similarly, in more recent international application WO 2014/114651 histidine is taught as the sole buffer component for formulating Adalimumab in a pharmaceutical composition while avoiding acetate and citrate.

Hence, only thanks to the diligent work and experiments performed in accordance with the present invention parameters sufficient and necessary to obtain a long-term stable antibody formulation based on a combined histidine buffer could be determined, *i.e.* the interplay of histidine with a second buffer component, in particular citrate and acetate, the pH value and the amount/concentration of the antibody, in particular Adalimumab in the formulation.

Accordingly, the present invention relates to a stable liquid aqueous pharmaceutical formulation which is free or substantially free of a phosphate buffer and comprises a therapeutically effective amount of Adalimumab in a buffered solution comprising L-Histidine and (i) citrate and/or (ii) acetate, preferably having a pH of about 5 to 5.5. Preferably, the pharmaceutical formulation of the present invention is selected from the group consisting of:
(a) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of aggregated species of Adalimumab after storage at 5°C for 3 to 6 months of about less than 5 %, see Example 5 and Figure 1A-C;
(b) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of aggregated species of Adalimumab after storage at 25°C for 3 to 6 months of about less than 5 %, see Example 5 and Figure 1D-F;
(c) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of acidic species of Adalimumab after storage at 25°C for 3 to 6 months of about less than 40%, see Example 7 and Figure 3D-F;
(d) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of aggregated species of Adalimumab under heat stress conditions at 55°C of about less than 5%, see Example 6 and Figure 2;
(e) a liquid aqueous pharmaceutical formulation comprising a therapeutically effective amount of Adalimumab in a buffered solution comprising L-Histidine and (i) citrate and/or (ii) acetate, said formulation having a pH of 5 to 5.5 and having a content of acidic species of Adalimumab under heat stress conditions at 55°C of about less than 40%, preferably less than 35%, see Example 8 and Figure 4;
(f) a liquid aqueous pharmaceutical formulation comprising a therapeutically effective amount of Adalimumab in a buffer solution comprising L-Histidine and (i) citrate and/or (ii) acetate, said formulation having a pH of 5 to 5.5 and having enhanced stability of at least 3 to 6 months at a temperature of about 5°C, 25°C and/or 55°C, see Examples 5 to 8 as well as Figures 1 to 4; and/or
(g) a liquid aqueous pharmaceutical formulation comprising a therapeutically effective amount of Adalimumab in a buffer solution comprising L-Histidine and (i) citrate and/or (ii) acetate, said formulation having a pH of 5 to 5.5 and retaining an activity in terms of TNFα neutralization of about 80%, preferably of about 85%, more preferably of about 90%, most preferably of about 95% after storage of 6 months at a temperature of about 5°C and/or 25°C, and/or after being subjected to heat stress at 55°C, freeze-thaw conditions at -20°C to 5°C and/or mechanical stress; see Example 9 as well as Figure 5.

The above mentioned features of the formulation of having a certain level of aggregated and/or acidic species of the antibody and/or having enhanced stability for about 3 to 6 months is to be determined according to the Examples and, unless indicated otherwise, in comparison to the reference formulation of Adalimumab shown in Table 6. With respect to the timeframe the feature may be fulfilled if the level of aggregated and/or acidic species of the antibody and/or enhanced stability of the formulation in absolute values and/or *vis-à-vis* the reference formulation is given for 3, 4, 5 and/or 6 months. Preferably, the feature is fulfilled for the 3-months term, most preferably in addition or alternatively for the 6-months term. Furthermore, the pharmaceutical formulation of the present invention may fulfil one, two, three, four, five or six of any one of the features (a) to (f) and optionally (g) in any combination or all of them. In addition, or alternatively the pharmaceutical formulation of the present invention, in particular with respect to feature (f) and/or (g) may display a stability profile which is virtually identical or highly similar to the profiles indicated in any one or all of Figures 1 to 5 for formulations with the ID NO (#0..): 7, 8, 9, 10, 11 or 12. The term "highly similar" means that the stability values of a pharmaceutical formulation of an anti-TNFa antibody, preferably Adalimumab based on a two component buffer system comprising histidine, when analyzed for any one of the above-mentioned features (a) to (f) and optionally (g), especially *vis-à-vis* the reference formulation in accordance with the appended Examples do not differ from the (relative) values for formulations with ID NO: 7, 8, 9, 10, 11 or 12 by more than 50%, preferably no more than 40%, more preferably no more than 30%, still more preferably no more than 20% and most preferably no more than 10% or 5%. It goes without saying that typically the values of a given candidate pharmaceutical antibody formulation are compared with those of a pharmaceutical formulation of ID NO: 7, 8, 9, 10, 11 or 12 which has the most ingredients in common with the candidate pharmaceutical formulation. In one embodiment, the pharmaceutical formulation of the present invention further comprises a polyol, preferably mannitol; a surfactant, preferably Polysorbate 80; and/or an alkali salt, preferably sodium chloride.

As used herein, "pharmaceutical formulation" refers to a formulation of a pharmaceutically active drug, such as a biologically active protein (*e.g.* anti-TNFa antibody Adalimumab) that is useful for treating a disease or disorder and which is suitable for parenteral administration (including but not limited to intravenous, intramuscular, or subcutaneous administration) and/or injection into a human patient in need thereof.

The "pharmaceutical formulation" as referred to in the present invention is prepared in such form as to permit the active ingredient to be effective, and includes only pharmaceutically acceptable excipients, diluents, and other additives deemed safe by regulatory authorities and which contains no additional components which are toxic to the subjects to which the formulation would be administered.

As used herein the phrase "liquid aqueous pharmaceutical formulation" refers to a pharmaceutical formulation as defined above in a liquid state using water as a solvent and comprising a pharmaceutically active drug in combination with one or more excipients. In a further embodiment the liquid aqueous pharmaceutical formulation of the present invention is a pharmaceutical formulation as defined above that maintains stability upon storage (e.g. chemical and/or physical stability/and/or biological activity) without the need for lyophilization, spray-drying, and/or freezing.

The phrases "free" or "substantially free" as used throughout the disclosure of the present invention indicate the exclusion of any recited elements or group of elements and the optional inclusion of other elements, of similar or different nature than the recited elements, that do not materially change the basic or novel properties of the specified dosage regimen, method, or composition.

In a pharmacological sense, in the context of the present invention, a "therapeutically effective amount" or "effective amount" of an antibody refers to an amount effective in the prevention or treatment of a disorder for the treatment of which the antibody is effective.

As used herein the term "Adalimumab" refers to the fully human recombinant monoclonal IgG1 antibody which binds to human TNF-alpha (hTNFα) and which is registered at Chemical Abstracts Service (CAS) under Registry Number 331731-18-1. Adalimumab consists of 1330 amino acids and has a molecular weight of 148 kDa which may vary depending on the glycosylation of the molecule. It is produced by recombinant DNA technology in Chinese hamster ovary (CHO) cells. It was derived from murine monoclonal antibody MAK195 using guided selection phage display. The fully human, affinity matured clone (clone ID: D2E7) comprises human-derived heavy and light chain variable regions, the amino acid sequences of which are disclosed in US patent No. 6,090,382 corresponding to international application WO97/29131 and a human IgG1 kappa constant region. Each IgG1 antibody molecule comprises two kappa light chains and two human IgG1 heavy chains. Each light chain consists of 214 amino acid residues and each heavy chain consists of 451 amino acid residues. Adalimumab binds specifically to TNFα and neutralizes the biological function of TNF by blocking its interaction with the p55 and p75 cell surface TNF receptors. Adalimumab is marketed by Abbott Laboratories US (now AbbVie Inc US) as liquid pharmaceutical formulation for subcutaneous administration under the trade name HUMIRA®; see also international application WO2004/016286. Adalimumab was approved by the US Food and Drug Administration (FDA) in 2002 and by the European Medicines Agency (EMA) in 2003 for the treatment of rheumatoid arthritis. Since then it received additional approval for the treatment of other TNF-mediated chronic inflammatory diseases, including psoriatic arthritis, polyarticular juvenile idiopathic arthritis, psoriasis, plaque psoriasis, ankylosing spondylitis (AS), axial spondyloarthritis, axial spondyloarthritis without radiographic evidence of AS, Crohn's disease, pediatric Crohn's disease and ulcerative colitis. Adalimumab can be used alone or in combination with methotrexate (MTX) or other non-biological disease modifying anti-rheumatic drugs (DMARDs). For the treatment of rheumatoid diseases, Adalimumab is typically administered by subcutaneous injection at 40 mg every one or two weeks. It is supplied in glass vials, prefilled glass syringes and as an autoinjection device (HUMIRA® Pen), as a sterile, preservative-free solution for subcutaneous administration. The solution of HUMIRA® is clear and colorless with a pH of about 5.2. The prefilled syringes and autoinjector (Pen) comprise 40 mg of Adalimumab in 0.8 mL of a buffered solution of mannitol, citric acid monohydrate, sodium citrate, disodium phosphate dihydrate, monosodium phosphate dihydrate, sodium chloride and Polysorbate 80.

Moreover, the term "Adalimumab" as used in the present invention encloses also proteins which are "biosimilar" to Adalimumab in accordance with the working definition declared in the published guidelines drafted by the FDA and the EMA which defines a biosimilar product to be one that is "highly similar" to a reference product despite minor differences in clinically inactive components ("Guideline on similar biological medicinal products containing biotechnology-derived proteins as active substance: quality issues (revision 1)", EMA/CHMP/BWP/247713/2012 as of 22.05.2014; "Guidance for Industry - Scientific Considerations in Demonstrating Biosimilarity to a Reference Product" (DRAFT GUIDANCE), U.S. Department of Health and Human Services, Food and Drug Administration as of February 2012). In practice there can be no clinically meaningful differences between the reference product and the biosimilar product in terms of safety, purity, and potency (Public Health Service (PHS) Act §262). In other words, a biosimilar form/protein of Adalimumab is an antibody which a regulatory authority deems to be "highly similar" to the reference product HUMIRA® on the basis of an abbreviated regulatory submission.

In one embodiment the biosimilar Adalimumab protein of the present invention and the reference product HUMIRA® utilize the same mechanism or mechanisms of action for the condition or conditions of use prescribed, recommended, or suggested in the proposed labeling, but only to the extent the mechanism or mechanisms of action are known for the active pharmaceutical ingredient of HUMIRA®.

In another embodiment, the condition or conditions of use prescribed, recommended, or suggested for the biosimilar Adalimumab protein of the present invention are the same as provided in the Summary of Product Characteristics (SMPC) and Label information for HUMIRA® as approved by the EMA and FDA, respectively. In one embodiment, the route of administration, the dosage form, and/or the strength of the biosimilar Adalimumab protein of the present invention are the same as recommended in the Summary of Product Characteristics (SMPC) and Label information for HUMIRA® as approved by the EMA and FDA, respectively.

As used in the disclosure of the present invention the term "reference product" refers to the FDA/EMA approved commercially available anti-TNFa antibody Adalimumab from Abbott/AbbVie which is marketed as "high concentration formulation" for subcutaneous administration under the trade name HUMIRA®. The liquid pharmaceutical formulation comprises 50 mg/mL Adalimumab buffered with citrate-phosphate (sodium dihydrogen phosphate dihydrate, disodium phosphate dihydrate, sodium citrate, citric acid monohydrate) at about pH 5.2 and contains further sodium chloride, mannitol, Polysorbate 80, and water for Injection; see international application WO2004/016286.

The terms "reference pharmaceutical formulation" or "reference formulation" as used in the disclosure of the present invention refer to a pharmaceutical formulation comprising anti-TNFa antibody Adalimumab formulated with a citrate-phosphate buffer system as disclosed in Example 1 (Table 6, Sample ID NOs #01, #02, #03) of the present invention. Accordingly, the term "reference buffer" as used throughout the present invention, refers to the buffer system comprising citrate and phosphate within composition and concentrations of the reference formulation as defined above.

The term "anti-TNFalpha antibody" or "anti-TNFa antibody" as used in the present invention refers to monoclonal antibodies and fragments thereof (Fab and or (Fab)₂ fragments, Fv fragments, single chain Fv (scFv) fragments and the like) as well as to Fc-Fusion proteins, directed against Tumor necrosis factor alpha (human TNFalpha, hTNFalpha, hTNFα, TNFα), a human cytokine which exists as a 17 kDa secreted form and a 26 kDa membrane associated form, the biologically active form of which is composed of a trimer of noncovalently bound 17 kDa molecules (Pennica D et al. 1984). In a particular embodiment the term "anti-TNFalpha antibody" or "anti-TNFa antibody" as used in the present invention refers to Adalimumab (CAS No. 331731-18-1) as defined by amino acid sequence of heavy and light chain variable regions as disclosed in US patent No. 6,090,382 and international application WO97/29131, respectively, and biosimilar forms/proteins of Adalimumab. For example, variants of anti-TNF antibodies such as Adalimumab which are said to show increased binding to the FcRn receptor or increased half-life compared to the native antibody are described in international application WO 2014/114651. Tumor necrosis factor alpha is involved in systemic inflammation processes, accountable for many of the clinical manifestations associated with autoimmune disorders such as rheumatoid arthritis, ankylosing spondylitis, inflammatory bowel disease, psoriasis, hidradenitis suppurativa, ulcerative colitis and refractory asthma. In a preferred embodiment, the concentration of the antibody is between 1-150 mg/mL, preferably between about 20-130 mg/mL, more preferably between about 30-100 mg/mL, even more preferred between 45 to 55 mg/mL. Most preferably, the concentration of the antibody is about 50 mg/mL.

Experiments performed in accordance with the present invention showed that a liquid aqueous pharmaceutical formulation comprising a therapeutically effective amount of Adalimumab in a buffer solution comprising L-Histidine and (i) citrate and/or (ii) acetate with a pH of 5 to 5.5 is capable of neutralizing the biological activity of TNFα of about 80%, preferably of about 85%, more preferably of about 90%, most preferably of about 95% after storage of 6 months at a temperature of about 5°C and/or 25°C, and/or after being subjected to heat stress at 55°C, freeze-thaw conditions at -20°C to 5°C and/or mechanical stress. Put in other words, Adalimumab formulated in a liquid aqueous pharmaceutical formulation of the present invention even after under various storage and stress conditions substantially retains its activity of reducing the biological activity of TNFα such as decribed in Example 9 to at least 80% or more compared to a control not subjected to said conditions or is even still able to nullify the biological activity of TNFα altogether. For example, the Histidine-Citrate buffer formulation without additional stabilizers (sample ID NO: #07) may be stored for 6 months at 5 °C or 25 °C and is still able to neutralize more than 90% of TNFα, whereas adding L-Arg (sample ID NO: #09) to the Histidine-Citrate buffer formulation further enhances its freeze-thaw stability and mechanical stess stability. Indeed, as can be gathered from Example 9 and Figure 5C, each Adalimumab formulation of the present invention is superior over the reference formulation of Adalimumab in at least one of the storage and stress conditions tested.

The storage and stress conditions including freeze-thaw conditions, heat stress conditions and mechanical stress, i.e. agitation studies are described in the Examples. For example, heat stress may be applied by subjecting the sample to a temperature of about 5°C and/or 25°C, and/or after being subjected to heat stress at 55°C for 10 minutes, the samples are frozen from 5°C to -20°C within 30 min and kept at -20°C for 1 hour, then thawed from -20°C to 5°C within 2 hours, with the freeze and thaw cycle being repeated 5 and 10 times, and the mechanical stress may be applied by shaking of the samples for 24 hours at 25°C on a planar shaker with a stirring speed of 400 RPM. Preferably, the concentration of Adalimumab in the test formulation is substantially the same as used in the Examples, i.e. 50 mg/mL. The TNFα neutralizing activity of a liquid aqueous pharmaceutical formulation of the present invention is preferably determined as illustrated in the Examples, i.e.by the effect of inhibiting cell death of L929 cells treated with TNFα and Actinomycin-D over time and/or in comparison to the reference formulation of Adalimumab shown in Table 6, whereas several dilutions of a liquid aqueous pharmaceutical formulation of the present invention are prepared as described in Example 9.

As used herein the term "excipient" encloses therapeutically inactive ingredients of the pharmaceutical formulation of the present invention. Depending on their individual properties, excipients may be included in a pharmaceutical formulation for a wide variety of purposes, for example, buffering agents to control pH, carbohydrates as bulking agents for lyophilization, polymers to increase solution viscosity, salts or sugars to stabilize proteins and act as tonicity agents to obtain physiological tonicity and osmolality, surfactants to inhibit protein adsorption to interfaces, preservatives to prevent microbial growth, anti-oxidants, cryoprotectants or diluents. A variety of formulation excipients have been shown to stabilize antibodies under different processing conditions and during storage, including sugars such as sucrose, lactose or dextrose, polyols (also known as sugar alcohols) such as mannitol or sorbitol, salts such as sodium chloride, amino acids such as arginine, histidine, lysine, aspartic acid, or glutamic acid, surfactants and water as solvent (Paborji M et al. 1994; Sarno MEC et al. 1999).

As used herein, the terms "buffer" or "buffered solution" refer to an aqueous solution consisting of a mixture of a weak acid and its conjugate base or a weak base and its conjugate acid that resists changes in pH. It is well known that both type and concentration of a buffering agent may have an impact on the stability of proteins (Wang W 1999). Therefore, the selection of the buffering agent is crucial to achieve pH control and desired stabilization of antibody formulations. Regarding antibody aggregation, the choice of a suitable buffer system and the pH play a critical role as it determines the net charge of the protein/antibody structure and subsequent electrostatic interactions (Chi EY et al. 2003) relative to increasing acidity or basicity of the solution. The increasing charge repulsion between the charged groups within the antibody in solution can destabilize its folded or native state (Vermeer AWP and Norde W 2000). Generally, weakly acidic conditions (pH 4.5-5.5) seem to be the optimum for most antibodies, since aggregation increases near neutral pH or near the isoelectric point (pI) (Moore JM et al. 1999, Szenczi A et al. 2006, Wan HZ et al. 2001). It is in general advisable to distinguish the pH value of the formulation by more than one unit from the protein's isoelectric point. IgG antibodies from mammals typically have relatively high pIs in the range of pH 6-9. Thus most formulation pH values are chosen in an acidic range (pH 4-6), in order to keep the antibody in a more soluble cationic state.

The liquid "high concentration formulation" (50 mg/mL) for subcutaneous administration of the anti-inflammatory drug Adalimumab currently on the market (HUMIRA®, AbbVie Inc, WO2004/016286) is buffered with citrate and phosphate at pH 5.2. The disadvantages of pharmaceutical formulations based on buffer systems comprising citrate and/or phosphate are well known in the art with a view to injection cite pain induced by citrate buffer (Kappelgaard A 2004, Frenken et al. 1993) and phosphate buffer (Fransson et al. 1996). In a few cases, phosphate buffer has been shown to be detrimental. For example, phosphate buffer accelerated deamidation of an IgG1 relative to citrate buffer at both pH 6.5 and 7.0 during storage at 25°C (Zheng JY and Janis LJ 2005). And as could be shown by Son and Kwon, 1995, about 38% of hEGF at 0.5 mg/l are deamidated in Tris-HCl at pH 7.0 on incubation at 60°C for 2 days, and the amounts of deamidated hEGF increase to 83, 63, 52, 51, and 49%, respectively, in buffers of PBS, sodium phosphate, sodium borate, sodium citrate, and sodium acetate (Son K and Kwon C 1995). Moreover, phosphate based-buffers seem to be a poor choice regarding their pH instability upon freezing (Kolhe P et al. 2010) since phosphate shows the greatest change in pH when going from 25-30°C. Whereas other buffers like histidine hydrochloride, sodium acetate, histidine acetate, citrate, and succinate showed less than 1 pH unit change (increase) over the temperature range from 25-30°C. The freeze-thaw instability of phosphate is concentration-dependent as shown for example in a study where freeze-thaw induced aggregation of a chimeric antibody (L6) was higher with increasing phosphate buffer concentration (0.01 vs. 0.05) (Paborji M et al. 1994).

Therefore, in a preferred embodiment, the liquid aqueous pharmaceutical Adalimumab formulation of the present invention is free or substantially free of a phosphate buffer.
The preferred buffer system of the present invention buffers the pH of the liquid aqueous pharmaceutical formulation in a range from about 5 to 8; preferably in the range from about pH 5 to about 5.5. Preferably, the pH is about 5.1 to 5.4, more preferably 5.15 to 5.3 and most preferably about 5.2 as illustrated for the formulations in the appended Examples. Thus, in a preferred embodiment, the liquid aqueous pharmaceutical Adalimumab formulation of the present invention has a pH of 5.2. Buffers that will control the pH in this range are well known in the art and commonly used in liquid formulations including acetate (e.g., sodium acetate), succinate (such as sodium succinate), gluconate, histidine, citrate and other organic acid buffers.

In a preferred embodiment, the liquid aqueous pharmaceutical Adalimumab formulation of the present invention is free or substantially free of a phosphate buffer and is buffered with L-Histidine in combination with either citrate or acetate.

In another preferred embodiment, the liquid aqueous pharmaceutical Adalimumab formulation of the present invention further comprises a polyol, a surfactant and sodium chloride.

A "polyol" according to the definition disclosed in the present invention is a substance with multiple hydroxyl groups, and includes sugars (reducing and nonreducing sugars), sugar alcohols and sugar acids. When it comes to the development of pharmaceutical antibody formulations with improved freeze-thaw stability, preferred polyols do not crystallize at freezing temperatures (e.g., -20°C) such that they destabilize the antibody in the formulation. The polyol may also act as a tonicity agent. Preferred polyols of the present invention are sugar alcohols such as mannitol, xylitol, erythritol, threitol, sorbitol and glycerol. A preferred polyol of the present invention is a sugar alcohol, and particularly mannitol. In one embodiment of the present invention the concentration of mannitol in the pharmaceutical formulation is 5 to 20 mg/mL. In a preferred embodiment of the present invention, the concentration of mannitol in the pharmaceutical formulation is 10 to 14 mg/mL, more preferably the concentration of mannitol in the pharmaceutical formulation is 12 mg/mL.

As used herein the terms "tonicity agent" or "isotonizing agent" or "tonicity modifier" or "tonifier" or "tonicifying agent" refer to an agent which functions to render a liquid aqueous pharmaceutical formulation similar in osmotic characteristics to physiologic fluids, in other words, the thus isotonic pharmaceutical formulation of interest has essentially the same osmotic pressure as human blood. Tonicity agents which are typically used include dextrose, mannitol, sodium chloride, potassium chloride and glycerin. Isotonic or physiologic formulations will generally have an osmotic pressure from about 275-325 mOsm. A preferred isotonizing agent comprised in the liquid aqueous pharmaceutical Adalimumab formulation of the present invention is sodium chloride. In one embodiment the concentration of sodium chloride in the pharmaceutical formulation of the present invention is 5 to 7.5 mg/mL, preferably 5.5 to 6.5 mg/mL sodium chloride. In a more preferred embodiment the concentration of sodium chloride in the pharmaceutical formulation of the present invention is about 6.2 mg/mL sodium chloride, and preferably 6.16 mg/mL sodium chloride.

In yet another preferred embodiment of the present invention, the pharmaceutical Adalimumab formulation further comprises a surfactant. There are basically two types of surfactants, nonionic and ionic. These surfactants drop surface tension of protein solutions and decrease the driving force for protein adsorption and or aggregation at hydrophobic surfaces. Nonionic surfactants are generally preferred in protein stabilization. Low concentrations of nonionic surfactants are often sufficient to prevent or reduce protein surface adsorption and/or aggregation due to their relatively low "critical micelle concentrations" (CMC) (Bam et al. 1995). The term "surfactant" as used in the present invention therefore particularly refers to nonionic surfactants which are widely used to stabilize proteins, suppress aggregation, and assist in protein refolding (Chi EY et al. 2003). Said surfactant is, preferably, a Polysorbate, which is an emulsifier derived from PEGylated sorbitan (a derivative of sorbitol) esterified with fatty acids. Polysorbate 80 and Polysorbate 20, also known as Tween 80 and Tween 20, respectively, have been widely incorporated in marketed protein pharmaceuticals at 0.0003-0.3% range. A preferred surfactant comprised in the pharmaceutical Adalimumab formulation of the present invention is Polysorbate 80 (polyoxyethylene(20)-sorbitan-monooleate). The preferred concentration range of Polysorbate 80 within the pharmaceutical Adalimumab formulation of the present invention is about 0.1-10 mg/mL. More preferably, the pharmaceutical Adalimumab formulation of the present invention comprises Polysorbate 80 at a concentration of about 0.5 to 1.5 mg/mL, mostly preferred to about 1 mg/mL of Polysorbate 80.

Hence, in accordance with the present invention a pharmaceutical formulation containing 5-20 mg/mL of mannitol, 0.1-10 mg/mL of Polysorbate 80 and 5 to 7.5 mg/mL sodium chloride is safe and well tolerated for use as a facilitating agent for the storage and administration of Adalimumab. Most preferably, the pharmaceutical formulation of the present invention contains about 12 mg/mL mannitol, about 1 mg/mL of Polysorbate 80 and about 6.2 mg/mL sodium chloride; see also the Examples.

In a further embodiment, the liquid aqueous pharmaceutical Adalimumab formulation of the present invention further comprises a stabilizer selected from L-Arginine and EDTA. The term "stabilizer(s)" as used herein refers to excipients which stabilize the native conformation of the protein, e.g. Adalimumab, respectively. Examples for protein stabilizers include polyols, sugars, amino acids, amines, and salts. Sucrose and trehalose are the most frequently used sugars and large polyols are in general better stabilizers than smaller polyols. Amino acids like arginine, histidine and glycine, methionine, proline, lysine, glutamic acid are among the preferred stabilizers of the present invention. Amino acids stabilize proteins by a variety of mechanisms including preferential exclusion, direct protein binding, buffering capacity, and antioxidant properties. L-Histidine for example has been used as a buffering agent for antibody formulations as described above and as an antioxidant that scavenges hydroxyl radicals in solution (Wade AM and Tucker HN, 1998).

Arginine has been widely used as a solubilizing agent during purification steps, as a mobile phase component in analytical HPLC, and as an excipient. L-Arginine (Arg) has been shown to be highly effective at suppressing aggregation. During refolding processes, arginine increases recovery by suppressing the aggregation of folding intermediates. Arginine also reduces protein aggregation during heat- or urea- induced denaturation, but it does not increase protein thermal stability. In addition, arginine has been found to increase protein solubility and reduce the viscosity of high concentration antibody formulations, thus allowing for shorter administration time. Despite a lack of understanding of the multiple mechanisms by which arginine interacts with affected protein solutions, due to its aggregation suppression ability and its safety in humans, the application of arginine in biologics is rapidly growing. Preferably, the pharmaceutical Adalimumab formulation of the present invention comprises L-Arginine (Arg) at a concentration of about 0.1 to 20 mg/mL, 0.2 to 15 mg/mL, 0.3 to 10 mg/mL, 0.4 to 7.5 mg/mL, and most preferably at a concentration of about 0.5 to 6.5 mg/mL.

The metal ion chelator Ethylenediaminetetraacetic acid (EDTA) is an aminopolycarboxylic acid which "captures" divalent metal ions thereby preventing complex formation of heavy metals, which otherwise would inactivate Adalimumab comprised in the pharmaceutical formulation of the present invention. Preferably, the pharmaceutical Adalimumab formulation of the present invention comprises EDTA (as used herein in form of its disodium salt, see also Examples 1 to 4) at a concentration of about 1 to 50 mg/mL EDTA, 5 to 15 mg/mL, 7.5 to 12.5 mg/mL, and most preferably at a concentration of about 10 mg/mL EDTA which is equivalent to a molar conctration of 29,74 mM.

In a preferred embodiment, the present invention relates to a liquid aqueous pharmaceutical Adalimumab formulation at a pH value in the range of 5 to 8, preferably of 5-5.5, which is free or substantially free of a phosphate buffer and comprises
(a) Adalimumab in a concentration of from 1 to 150 mg/mL, 20 to 130 mg/mL, 30 to 100 mg/mL, preferably of about 50 mg/mL;
(b) mannitol in a concentration of about 10-14 mg/mL, preferably 12 mg ±0,5 mg;
(c) Polysorbate 80 at a concentration of about 0.1-5 mg/mL;
(d) L-Histidine-HCl in a concentration of 0.1-0.5 mg/mL and L-Histidine base in a concentration of 0.01-0.025 mg/mL, preferably together with
   (i) citric acid monohydrate in a concentration of 0.1-1.5 mg/mL and trisodium citrate dihydrate in a concentration of 0.25-3.5 mg/mL and/or
   (ii) sodium acetate trihydrate in a concentration of 0.1-0.5 mg/mL and acetic acid in a concentration of 0.01-0.05 mg/mL; and
(e) sodium chloride in a concentration of 6.0-6.4 mg/mL; optionally further comprising a stabilizer selected from (f) L-Arginine or (g) EDTA.

As mentioned above and illustrated in the Examples, liquid aqueous pharmaceutical formulations of Adalimumab which are free or substantially free of a phosphate buffer and which are formulated in L-Histidine-based buffer systems provide suitable alternative pharmaceutical Adalimumab formulations with increased long term stability in terms of aggregate formation and generation of acidic species. More specifically, the phosphate-buffer free pharmaceutical formulations of Adalimumab which were buffered with Histidine-citrate or Histidine-acetate buffer of the present invention show improved stability upon storage at 25°C over a period of at least three to six months compared to a citrate-phosphate buffer based reference pharmaceutical Adalimumab formulation composed according to the formulation of marketed product HUMIRA®. In particular, the Histidine-citrate and Histidine-acetate buffered Adalimumab formulations of the present invention exhibit less aggregate formation as determined by size exclusion chromatography even when the formulation has been stored for up to six months at 25°C ± 3°C as demonstrated in Example 5 and Fig. 1D-F as well as upon heat stress of 55°C as shown in Example 6 and Fig. 2 compared to the citrate-phosphate buffered reference pharmaceutical formulation.

In addition, the Histidine-citrate or Histidine-acetate buffered Adalimumab formulations of the present invention showed decreased deamidation with a view to less formation of acidic species as determined by strong cation exchange chromatography even when the formulation has been stored for up to six months at 25°C ± 3°C as demonstrated in Example 7 and Fig.
3D-F as well as upon heat stress of 55°C as shown in Example 8 and Fig. 4 compared to the citrate-phosphate buffered reference pharmaceutical formulation.

The phrase "stable pharmaceutical formulation" according the present invention is a pharmaceutical formulation wherein the antibody of interest e.g. Adalimumab retains its physical stability and/or chemical stability and/or biological activity upon storage at the intended storage temperature (*e.g.,* 5°C and/or 25°C) as required by a regulatory agency for its approval. For example, a stable pharmaceutical composition is a formulation that between the time that it is made and the time that it is used (or reaches the end of its intended shelf-life), does not undergo any changes in its physical, chemical or biological properties which renders it unsafe or ineffective for its intended pharmaceutical use according the standards established in ICH Q5C, "Quality of Biotechnological Products: Stability Testing of Biotechnological/Biological Products" by the International Conference on Harmonization of Technical Requirements of Pharmaceuticals for Human Use. Stability of a protein *e.g.,* Adalimumab can be determined by detecting and quantifying chemically altered forms of the protein. The amount of monomeric antibodies in the pharmaceutical formulation is a main qualitative feature for the determination of a suitable pharmaceutical formulation. Since aggregates are a leading cause for (severe) side effects, the content of monomers displays the actual pharmaceutically active amount of the drug, *i.e.,* the antibody. As discussed above this is particularly true for pharmaceutical formulations for self-administration which are at the risk of improper storage conditions such at high temperatures.

The liquid aqueous pharmaceutical formulation(s) or buffer(s) with "enhanced stability" or which are "more stable" compared to a reference pharmaceutical formulation refers to liquid aqueous pharmaceutical formulation(s) or buffer(s) wherein the protein, i.e. antibody denotes quantitative differences between two states (e.g. physical stability and/or chemical stability and/or biological activity of the protein *e.g.* the antibody), referring to at least statistically significant differences between the two states.

As used herein, the term "aggregation" incorporates both the terms "protein aggregation" and "antibody aggregation" and refers to the formation of protein species of higher molecular weight, such as oligomers or multimers, instead of the desired defined species of the biopharmaceutical drug (*e.g.* a monomer). Protein aggregation is thus a universal term for the formation of all kinds of not further defined multimeric species (aggregated species) that are formed by covalent bonds or noncovalent interactions. Protein aggregation is one of the three most common protein degradation pathways besides deamidation, and oxidation (Cleland JL et al. 1993). Protein aggregation in solution is primarily caused by the solvent environment (pH, salt, cosolvents, etc.), temperature, or surface interactions. Aggregation problems have been implicated in adverse reactions and other safety issues since the beginning of clinical applications of protein pharmaceuticals. Immunoglobulin aggregates have long been known to cause anaphylactic reactions (Cleland JL et al. 1993, Carpenter JF et al. 1999). To minimize such risks from therapeutic proteins in clinical applications, there is a need for the optimization of pharmaceutical formulations to reduce aggregation during storage, handling, and shipping (Remmele RL et al. 2006).

The term "deamidation" as used herein refers to the deamidation of asparagine residues of the anti-TNFa antibody Adalimumab of the present invention. Deamidation is a prevalent protein degradation pathway and normally, purified antibody preparations contain many acidic forms. Storage can easily generate large amounts of acidic products. Deamidation of asparagine residues is one of the most common post-translational modifications occurring in therapeutic proteins produced using recombinant DNA technology. Deamidation of the protein results in the conversion mainly of an asparagine residue to a mixture of isoaspartate and aspartate. Deamidation of glutamine residues occur also but to a lesser extent (Chelius D et al. 2005).

As used herein, the terms "acidic species", "acidic impurity (impurities)" and "acidic variant(s)" refer to a variant or variants of a target protein which is more acidic (*e.g.* as determined by strong cation exchange chromatography (SCX-HPLC)) than the target protein. An example of an acidic species/impurity/variant is a deamidated species/impurity/variant.

In another preferred embodiment the pharmaceutical formulation of the present invention is suitable for single use subcutaneous injection and contained in a pharmaceutical container. Subcutaneous administration is the preferred route of administration in therapeutic regimens which require repeated treatment, as is the case in many chronic diseases such as autoimmune diseases (*e.g.,* rheumatoid arthritis, ankylosing spondylitis, inflammatory bowel disease, psoriasis, hidradenitis suppurativa, ulcerative colitis and refractory asthma) where medication eventually has to be administered life-long and where home (self) medication is desirable with respect to patient compliance and cost reduction.

As shown in Examples 1 to 9 and illustrated in Figures 1 to 5 in pharmaceutical formulations of the present invention, wherein the composition is free or substantially free of a phosphate buffer, the presence of ingredients other than the therapeutic antibody (a) and the components (b) a polyol; (c) a surfactant; (d) a buffer component comprising L-Histidine and (i) citrate and/or (ii) acetate; and (e) sodium chloride; and optionally (f) L-Arginine and/or (g) EDTA further described above is dispensable. Therefore, in one embodiment the pharmaceutical formulation of the present invention is free or substantially free of dicarboxylic acids, monosaccharides, oligosaccharides and/or additional amino acids. In a particularly preferred embodiment the pharmaceutical formulation of the present invention is an aqueous formulation which consists of components (a) to (e) optionally together with (f) and/or (g) with the further specifications for these features disclosed hereinbefore and illustrated in the Examples, respectively.

In another aspect the present invention relates to a prefilled pharmaceutical container comprising any one of the liquid aqueous pharmaceutical formulations being formulated according to the first aspect of the present invention disclosed hereinbefore and illustrated in the Examples, respectively.

In a preferred embodiment, the prefilled pharmaceutical container of the present invention comprises a liquid aqueous pharmaceutical formulation having a pH of 5 to 5.5, wherein the composition is free or substantially free of a phosphate buffer and comprises
(a) Adalimumab;
(b) a polyol;
(c) a surfactant;
(d) a buffer component comprising L-Histidine and (i) citrate and/or (ii) acetate;
(e) sodium chloride; see also *supra.*

In one embodiment of the present invention, the above mentioned composition in the container further comprises (f) L-Arginine and/or (g) EDTA.

In one embodiment of the present invention, the prefilled pharmaceutical container is a prefilled syringe, injection pen, ampoule, bottle, autoinjector or an infusion bag which comprises the liquid aqueous pharmaceutical formulation according to the first aspect of the invention. Preferably, the container is closed under nitrogen protective atmosphere.

As used herein, the term "protective atmosphere" refers to such a protective atmosphere with increased nitrogen content. Protective gas atmospheres (also known as protective/modified atmosphere or protective/modified gas) are commonly used for extending the shelf life of products, such as foods or medicaments/pharmaceuticals in a gas-tight packaging to slow or even stop microbiological, enzymatic and biochemical adverse physical processes that lead to the rapid destruction of the good during storage. Typically used as protective gas are gas mixtures of carbon dioxide and nitrogen wherein carbon dioxide exhibits an inhibitory effect on the growth of microorganisms such as bacteria and fungi, while nitrogen gas primarily serves as a support to prevent the collapse of the package. Furthermore, in most protective gas atmospheres the oxygen concentration is reduced in comparison to air, to prevent growth of microorganisms.

In a further preferred embodiment of the invention, the dose strength of Adalimumab as provided within the pharmaceutical formulation is preferably 20, 40, 80, 120 or 160 mg. Preferably, the different dose strengths of Adalimumab are provided within the container, preferably within the prefilled syringes of the present invention, wherein the different dose strengths in the prefilled syringes are 20, 40 and/or 80 mg, preferably having a dose strength of Adalimumab of 20 or 80 mg, and preferably wherein Adalimumab is designed to be administered at a total dose of 20, 40 or 80 mg.

In a further preferred embodiment, the present invention provides a set, *e.g.* kit of at least two containers, wherein the containers have different dose strengths of Adalimumab, preferably 20 and 80 mg. In a further embodiment, the different dose strengths are constituted by different filling volumes of the same liquid aqueous pharmaceutical Adalimumab formulation. In yet a further embodiment, the different dose strengths in the prefilled syringes are 20, 40, 80, and/or 160 mg. Preferably, the set of the present invention, e.g. kit comprises at least one container with a dose strength of 20, 80, and/or 160 mg.

*Hitherto,* the current total dose of Adalimumab was 40 mg for treating adult patients suffering from rheumatoid arthritis (RA), ankylosing spondylitis (AS), or psoriatic arthritis (PsA). However, for pediatric patients (2 to < 4 years old) suffering from juvenile idiopathic arthritis (JIA) a total dose of 20 mg is recommended, which can be increased to a maximum of 40 mg. The recommended induction dose for pediatric patients (< 40 kg body weight) suffering from Crohn's Disease (CD) includes 40 mg Adalimumab followed by a maintenance dose of 20 mg of the antibody. In contrast, the recommended dose for pediatric patients (≥ 40 kg body weight) suffering from severe CD is 80 mg of Adalimumab, followed by 40 mg of Adalimumab. In order to achieve a faster response to the therapy of severe CD, an induction dose of 160 mg followed by a maintenance dose of 80 mg of Adalimumab may be chosen. Accordingly, in one embodiment the dose strength of Adalimumab in the prefilled syringes is preferably 20 mg or an integral multiply thereof in the accompanying container, preferably 2 x 20 mg, 4 x 20 mg or 8 x 20 mg.

The recommended Adalimumab dose regimen for adult patients with ulcerative colitis is 160 mg initially on day 1, followed by 80 mg on day 15. On day 29 a maintenance dose of 40 mg every other week is given. The recommended dose of Adalimumab for adult patients with plaque psoriasis (Ps) is an initial dose of 80 mg, followed by 40 mg given every other week starting one week after the initial dose. Accordingly, in another embodiment the dose strength of Adalimumab in the prefilled syringes is preferably 40 mg or an integral multiply thereof in the accompanying container, preferably 2 x 40 mg, 4 x 40 mg.

Furthermore, the set of containers of the present invention, in particular with a dose strength including 20 mg, 80 mg, 120 mg and/or 160 mg as well as optionally comprising 40 mg in addition are advantageous for peer groups of patients who are in need of different dose regimen but are used to receive their therapy jointly or under a common control by a physician. In addition, the low 20 mg dose strength turned out be useful for patients who suffer from skin irritations when using a higher dose strength so that they prefer receiving a low dose twice. On the other hand, some patients show preference for a high dose strength of 80, 120 or even 160 mg in case they are for example a couple or friends who wish receiving Adalimumab therapy from the same batch, *i.e.,* syringe or pen. Hence, the set of containers of the present invention with different dose strengths of Adalimumab supplies a need of patients, which hitherto had not been recognized, and in view of the applicability for joint treatment may help to overcome the patient's fear against the treatment, make therapy more comfortable for the patients. In addition, since the set of containers can be supplied in different compositions of dose strengths adapted to the patient or group of patients the therapy will reduce *inter alia* costs since there will be less excess of the drug, if any compared to a set of single dose strengths of 40 mg and thus also less waste and contamination of the environment.

The term "disorder" as used in the present invention, is any condition that would benefit from treatment with the antibody, *i.e.,* Adalimumab, respectively. This includes chronic and acute disorders or diseases including those pathological conditions which predispose the subject to the disorder in question. According one embodiment, the present invention provides a liquid aqueous pharmaceutical formulation, *e.g.,* in a container or set of containers as defined above for use as a medicament for use in "the treatment of a disorder in which TNFα activity is detrimental". Such disorders include but are not limited to sepsis, infectious diseases, autoimmune diseases, transplant rejection, malignancy, pulmonary disorders, cardiac disorders, intestinal disorders, graft- versus-host disease, diseases of the nervous system and the like.

Autoimmune diseases which are suitable for treatment with the liquid aqueous pharmaceutical Adalimumab formulation of the present invention, *e.g.,* in a container or set of containers as defined above include but are not limited to arthritic and rheumatic diseases, like rheumatoid arthritis (RA), psoriatic arthritis (PsA), polyarticular juvenile idiopathic arthritis, psoriasis, plaque psoriasis (Ps), ankylosing spondylitis (AS), axial spondyloarthritis, axial spondyloarthritis without radiographic evidence of AS, Crohn's disease (CD), pediatric Crohn's disease and ulcerative colitis.

Inflammatory diseases which are suitable for treatment with the liquid aqueous pharmaceutical Adalimumab formulation of the present invention, *e.g.,* in a container or set of containers as defined above include but are not limited to inflammatory bone disorders and bone resorption disease, hepatitis, including alcoholic hepatitis and viral hepatitis, coagulation disturbances, reperfusion injury, keloid formation, scar tissue formation, pyrexia, periodontal disease, obesity and radiation toxicity.

Suitable infectious diseases are viral and/or bacterial infections. Suitable diseases of the nervous system are, among others, neurodegenerative disorders like Parkinson's disease, Alzheimer's disease, multiple sclerosis, Huntington's disease, or Amyotrophic lateral sclerosis.

In a more preferable embodiment the liquid aqueous pharmaceutical formulation (and the container or set of containers for use in accordance with the present invention) is designed to be administered subcutaneously to the human subject in need thereof on a biweekly dosing regimen of every 13-15 days. Preferably, the container or set of containers for use in accordance with the present invention has a dose strength of 20 or 80 mg, preferably wherein Adalimumab is designed to be administered at a total dose of 20, 40 or 80 mg.

In a preferable embodiment the liquid aqueous pharmaceutical formulation of the present invention is intended for administration in combination with an additional therapeutic agent that inhibits TNFα production or activity, preferably but not limited to methotrexate.

Examples 1 to 9 which follow and corresponding Figures 1 to 5 further illustrate the invention, but should not be construed to limit the scope of the invention in any way. Detailed descriptions of conventional methods, such as those employed herein can be found in the cited literature.

### EXAMPLES

Several formulations comprising alternative buffer systems for the citrate-phosphate buffered market formulation of Adalimumab exist, comprising mono-or dicarboxylic acids or salts thereof together with an amino acid or polyol stabilizer (see, *e.g.,* international application WO2012/089778), buffers selected from histidine-buffers, arginine-buffers, succinate-buffers, citrate-buffers or acetate-buffers and stabilizers selected from amino acids and cyclodextrines (see, e.g., international applications WO2013/164837 and WO2014/039903), or formulations of Adalimumab which are buffered at a different pH (at least 5.5, preferably of 6.25 +/- 0.5) like in international application WO2013/186230. However, in the light of the requirements of a stable liquid pharmaceutical formulation with particular regard to the suitability for life-long self-administration and for the full exploitation of the clinical potency of an antibody like in the case of Adalimumab, there is still a need for the development of further liquid aqueous pharmaceutical formulations of Adalimumab with improved storage stability.

For the screening of buffer systems suitable as alternatives superior over those for the citrate-phosphate buffered Adalimumab formulation available on the market, further Adalimumab pharmaceutical formulations were prepared within 4 different buffer systems: a) citrate (mono buffer system), b) histidine-citrate, c) histidine-acetate and in d) citrate-phosphate (reference buffer). For the evaluation of the effect of additional stabilizers, the four alternative Adalimumab formulations of the present invention additionally included one of the stabilizers L-Arginine or EDTA. All the other ingredients (sodium chloride, mannitol, Polysorbate 80) were maintained qualitatively and quantitatively according to the Adalimumab formulation of the marketed product HUMIRA®. Stability of the different Adalimumab formulations was tested during long-term storage at 5°C and 25°C over a period of 6 months and under stress conditions like heating towards 55°C. A summary of all tested formulations is provided within table Table 1.

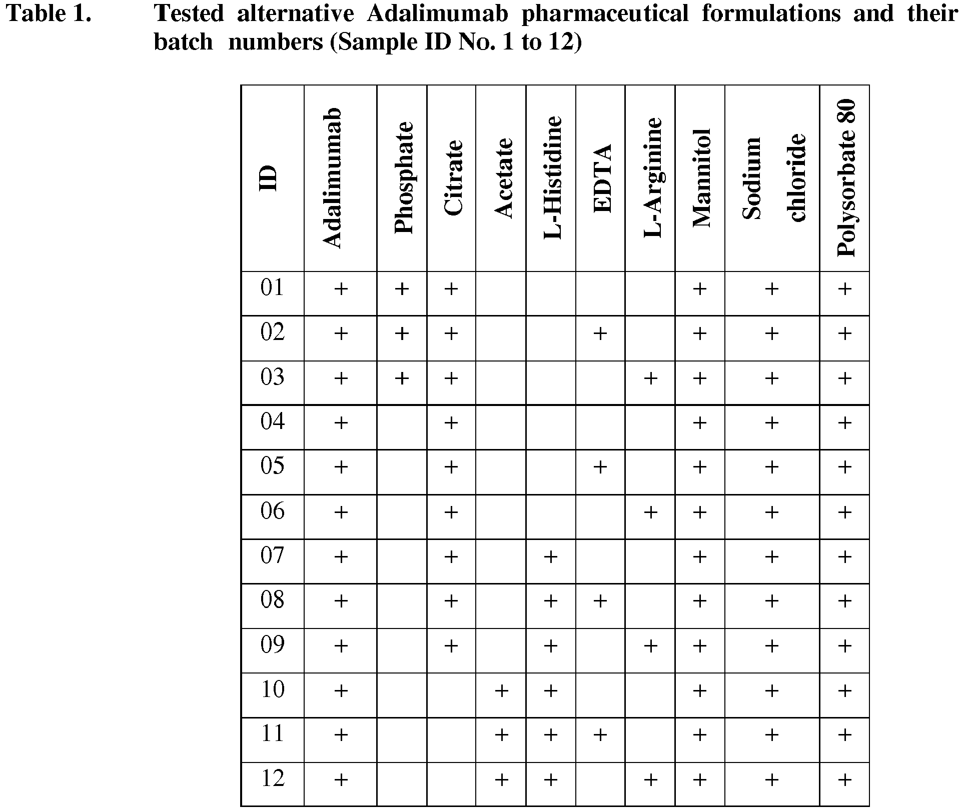

The experiments comprising the used material and the methods performed according to the present invention and their results are summarized in the following.

**Table 2. Equipment**

| **Equipment** | **Manufacturer** | **Model** | **Serial Number** |
|---|---|---|---|
| UHPLC | Shimadzu Corp. | Nexera | L20234975345 |
| pH meter | Radiometer | PHM220 | 657R012N015 |
| HPLC | Agilent Biosystems Inc. | 1200 | DE63059747 |

**Table 3. HPLC Columns**

| **Column** | **Manufacturer** | **Part Number** | **Lot Number** |
|---|---|---|---|
| Acquity UPLC BEH300 C18 1.7µm 2.1×100mm | Waters Corp. | 186003686 | 0124332941 |
| Yarra 3u SEC-3000 300×4.6mm | Phenomenex | 00H-4513-E0 | 5623-17 |
| YMC-BioPro SP-F 100×4.6mmI.D. S-5µm | YMC | SF00S05-1046WP | 10183 |

**Table 4. List of Materials/Reagents**

| **Material/Reagents** | **Manufacturer** | **Part Number** | **Lot number** |
|---|---|---|---|
| 2-(N-morpholino)ethanesulfonic acid monohydrate BioUltra | Fluka | 69889 | BCBK9895V |
| 2-(N-morpholino)ethanesulfonic acid sodium salt BioPerformance | Sigma | M3058 | SLBB6703V |
| Sodium chloride BioXtra | Sigma-Aldrich | S7653 | SLBC3215V |
| Potassium phosphate dibasic, for HPLC, ≥99.0% | Fluka | 17835 | AM0419904327 |
| Potassium phosphate monobasic, for HPLC, ≥99.5% | Fluka | 60221 | B0691108221 |
| Potassium chloride, BioXtra, ≥99.0% | Sigma-Aldrich | P9333 | BCBK9830V |
| Phosphate buffered saline | Sigma | P4417 | SLBJ2117V |

**Table 5. List of Chemicals**

| **Name** | **Manufacturer/Supplier** | **Purity** | **Lot Number** |
|---|---|---|---|
| L-Arginine (free base) | Merck KGaA | 99,8% | k42667842150 |
| Acetic acid (100%) (IN) | Celanese Gmbh | 100 % | R46162199 |
| Citric acid monohydrate | Jungbunzlauer | 100,2% | 1135667 |
| Disodium hydrogen phosphate dodecahydrate | Merck KGaA | 100,4% | K93244173 |
| Disodium EDTA (anhydrous) | Merck KGaA | 99,3% | D00104170 |
| Hydrochloric Acid | | | |
| L-Histidine base | Ajinomoto | 98,5% | R016E013 |
| L-Histidine HCl | Merck KGaA | 100,1% | K43128954 |
| Mannitol | Roquette Freres | 98,9% | E636Y |
| Polysorbate 80 | Croda Chocques SAS | - | 1702NP3483 |
| Sodium acetate trihydrate high grade | Merck KGaA | 99,5% | A0337312 |
| Sodium chloride | Salinen Austria AG | 99,97% | CRS171212 |
| Sodium dihydrogen phosphate dihydrate | Honeywell Speciality Chemicals Seelze Gmbh | 100,0% | C0130 |
| Trisodium citrate dihydrate | Honeywell Speciality Chemicals Seelze Gmbh | 100,4% | A3330 |

### Production/Purification of Adalimumab

The active pharmaceutical ingredient utilized herein was Adalimumab which was produced in a clone derived from CHO cells (DG44) and purified by protein A affinity chromatography, cation exchange chromatography (CEX) and anion exchange chromatography (AEX) steps. After the last purification step, the antibody was concentrated to a final concentration of about 50 mg/mL in the desired buffer and stored at a temperature below -60°C.

### Preparation of the pharmaceutical Adalimumab formulations

Adalimumab was purified by protein A antibody affinity chromatography and then cation and anion exchange chromatography was used during the downstream process. After these purification steps, different formulations were prepared by diafiltration. The different diafiltrated solutions were prepared using buffers containing mannitol and sodium chloride, while Arginine, EDTA and Tween 80 stabilizing excipients were added in subsequent steps. After diafiltration the protein solution was concentrated to approximately 65 mg/mL. Tween 80 was added from concentrated solution (10 times concentrated) containing the same buffer solutions with mannitol and the sodium chloride. The protein concentration and the pH were measured in the obtained formulation samples. The target concentration of the samples was 55 ± 2.5 mg/mL. The protein concentration and the pH of the samples were determined, and then the solutions were filtered through a 0.22-µm pore size sterile filter into single use sterile polycarbonate tubes.

Arginine or EDTA excipients were added to the buffer solutions containing mannitol and sodium chloride. The different pharmaceutical formulations were prepared by diluting the protein solution to 50 mg/mL. The protein solution was diluted with the different buffer solutions only or together with the EDTA- or L-Arginine - containing buffer solutions. If the concentration of the samples was out of the desired range it was adjusted by diluting to the target concentration.

The prepared samples were filtered through a 0.22-µm pore size sterile filter. The aliquots of the samples were transferred into sterile centrifuge tubes using sterile equipment under laminar air flow and the tubes were closed under nitrogen gas flow. The Adalimumab formulation samples were then ready for the stability experiments.

### Long term stability Studies

The different Adalimumab formulation samples were stored for 6 months under 5°C ± 3°C and 25°C ± 2°C. Samples were analyzed after 1, 2, 3, and 6 months.

### Freeze-Thaw Conditions

The freeze thaw samples were prepared on the day of analysis to match with t=0. Freeze-thaw stress of the samples was applied in a Telstar Liobeta 15 freeze drying instrument. The samples were frozen from 5°C to -20°C within 30 min and kept at -20°C for 1 hour. The samples were thawed from -20°C to 5°C within 2 hours. The freeze and thaw cycle was repeated 5 and 10 times, respectively, for each sample.

### Heat Stress Conditions

Heat stress was applied by incubation of the samples for 10 min at 55°C, a temperature near the melting point of the protein.

### Agitation Studies

Mechanical stress was applied by shaking of the samples for 24 hours at 25°C on a planar shaker with a stirring speed of 400 RPM.

### One month storage at elevated temperature

The samples were stored at 40 ± 2 °C for 1 month with a relative humidity of 75 ± 5 %. The mechanically stressed, freeze-thawed and high temperature stressed samples were stored for an additional month at 25 ± 2 °C and analyzed again.

### Protein content by RP-UHPLC-UV₂₈₀

Reversed Phase Ultra-High Performance Liquid Chromatography with UV detector (RP-UHPLC-UV) using absorbance at 280 nm was used to measure the concentration of Adalimumab within the different formulation samples.

### pH Measurements

The pH of each sample was measured using a micro-pH probe. Before the start of analysis the pH probe was calibrated with three pH standards. The pH values of the stability samples were measured by transferring 500µL of each stability sample to 1000 µL PCR tube. The micro-pH probe was then submerged into the sample and after the value stabilized it was recorded.

### Size Exclusion Chromatography (HP-SEC)

Stability of the alternative Adalimumab formulations with respect to purity/aggregation and fragmentation was measured using Size Exclusion High Performance Liquid Chromatography (HP-SEC) which separates molecules based on size. The early eluting peak corresponds to high molecular weight species or % aggregates. The main peak (intact protein) corresponds to % monomer. The late eluting peak corresponds to low molecular weight species or % fragments. SEC method parameters are briefly summarized below:
∘ Sample preparation: 50× dilution with water
∘ Column Information: Yarra 3u SEC-3000 300×4.6mm
∘ Analysis Buffer: 200 mM potassium phosphate buffer (pH ≈ 7.0) including 100 mM potassium chloride
∘ Flow rate: 0.35 mL/min
∘ Column temperature: 30°C
∘ Detection: UV, λ= 215 nm
∘ Injection volume: 2 µl
∘ Sample temperature: 5°C

SEC data was analyzed using "Empower2" software based on relative % areas of main, aggregated and fragmented peaks.

### Strong Cation Exchange (SCX) Chromatography (SCX-HPLC)

Charge heterogeneities and post translational modifications of intact digested Adalimumab formulation samples were analyzed by Strong Cation Exchange (SCX) High Performance Liquid Chromatography (SCX-HPLC).
SCX chromatography method parameters are briefly summarized below:
Intact measurement:
∘ Sample preparation: 50× dilution with water
∘ Column Information: YMC-BioPro SP-F 100×4.6mmI.D. S-5µm
∘ Mobile phase A: 10 mM MES buffer (pH ≈ 6,0)
∘ Mobile phase B: 200 mM sodium chloride in mobile phase A
∘ Flow rate: 0.85 mL/min
∘ Column temperature: 25°C
∘ Detection: Fluorescence, λ_{EX} = 280 nm, λ_{EM} = 350 nm
∘ Injection volume: 5 µl
∘ Sample temperature: 5°C
∘ Gradient program:

| Time [min] | Mobile phase A [%] | Mobile phase B [%] |
|---|---|---|
| 0 | 56.6 | 43.4 |
| 18 | 33.6 | 66.4 |
| 19 | 33.6 | 66.4 |
| 19,5 | 56.6 | 43.4 |
| 25 | 56.6 | 43.4 |

The following non-limiting Examples represent several alternative Adalimumab formulations which exemplify the present invention.

### Example 1: Adalimumab pharmaceutical formulation comprising Citrate-Phosphate buffer (reference formulation)

Adalimumab was purified according techniques well known in the art. In this Example, the purified Adalimumab was formulated in the presence of citrate-phosphate buffer along with mannitol, sodium chloride and Polysorbate 80 with or without (#01) one of the additional stabilizers EDTA (#02) and L-Arginine (#03) at concentrations as shown in Table 6. pH of the formulation was adjusted to about pH 5.2. Excipients were added to the protein solution from respective stock solutions to adjust the final concentration and the volume was filled up to the desired level with sterile water or Water for Injection. The formulated bulk was distributed in a suitable container (like vials, syringes etc.) for storage under normal and stress conditions. Stability with regard to aggregate formation and deamidation/formation of acidic impurities (species) of the respective formulations was measured at different time points by HP-Size exclusion chromatography and upon heat exposure by HP-SCX, respectively. Results are shown in Figures 1 to 4.

**Table 6: #01 Citrate-phosphate buffer (reference formulation)*.**

| **Components** | **Concentration** | |
|---|---|---|
| | **mg/mL** | **mmol/L** |
| Adalimumab | 50.00 | |
| Sodium chloride | 6.16 | 105.45 |
| Sodium dihydrogen phosphate dihydrate | 0.86 | 5.53 |
| Disodium hydrogen phosphate dodecahydrate | 3.07 | 8.57 |
| Trisodium citrate dihydrate | 0.30 | 1.02 |
| Citric acid monohydrate | 1.30 | 6.19 |
| Mannitol | 12.00 | 65.87 |
| Polysorbate 80 | 1.00 | |
| *± *10.00 mg*/*mL Disodium EDTA* (#02) *or ± 4.36 mg*/*mL L-Arginine free base (#03)* | | |

### Example 2: Adalimumab formulation comprising Citrate buffer (mono-buffer)

Adalimumab was purified according techniques well known in the art. In this Example, the purified Adalimumab was formulated in the presence of citrate buffer as mono buffer system along with mannitol, sodium chloride and Polysorbate 80 with or without (#04) one of the additional stabilizers EDTA (#05) and L-Arginine (#06) at concentrations as shown in Table 7. pH of the formulation was adjusted to about pH 5.2. Excipients were added to the protein solution from respective stock solutions to adjust the final concentration and the volume was made up to the desired level with sterile water or Water for Injection. The formulated bulk was distributed in a suitable container (like vials, syringes etc.) for storage under normal and stress conditions. Stability with regard to aggregate formation and deamidation/formation of acidic impurities (species) of the respective formulations was inspected at different time points by HP-Size exclusion chromatography and upon heat exposure by HP-SCX, respectively. Results are shown in Figures 1 to 4.

**Table 7: #04 Citrate buffer*.**

| **Components** | **Concentration** | |
|---|---|---|
| | **mg/mL** | **mmol/L** |
| Adalimumab | 50.00 | |
| Sodium chloride | 6.16 | 105.45 |
| Trisodium citrate dihydrate | 3.17 | 10.77 |
| Citric acid monohydrate | 0.83 | 3.97 |
| Mannitol | 12.00 | 65.87 |
| Polysorbate 80 | 1.00 | |
| *± *10.00 mg*/*mL Disodium EDTA* (#05) *or* ± *4.36 mg*/*mL L-Arginine free base (#06)* | | |

### Example 3: Adalimumab pharmaceutical formulation comprising Histidine-Citrate buffer

Adalimumab was purified according techniques well known in the art. In this Example, the purified Adalimumab was formulated in the presence of Histidine-Citrate buffer along with mannitol, sodium chloride and Polysorbate 80 with or without (#07) one of the additional stabilizers EDTA (#08) and L-Arginine (#09) at concentrations as shown in Table 8. pH of the formulation was adjusted to about pH 5.2. Excipients were added to the protein solution from respective stock solutions to adjust the final concentration and the volume was filled up to the desired level with sterile water or Water for Injection. The formulated bulk was distributed in a suitable container (like vials, syringes etc.) for storage under normal and stress conditions. Stability with regard to aggregate formation and deamidation/formation of acidic impurities (species) of the respective formulations was inspected at different time points by HP-Size exclusion chromatography and upon heat exposure by HP-SCX, respectively. Results are shown in Figures 1 to 4.

**Table 8: #07 Histidine-Citrate buffer*.**

| **Components** | **Concentration** | |
|---|---|---|
| | **mg/mL** | **mmol/L** |
| Adalimumab | 50.00 | |
| Sodium chloride | 6.16 | 105.45 |
| L-Histidine base | 0.02 | 1.11 |
| L-Histidine HCl | 0.24 | 11.20 |
| Trisodium citrate dihydrate | 1.58 | 5.38 |
| Citric acid monohydrate | 0.42 | 1.98 |
| Mannitol | 12.00 | 65.87 |
| Polysorbate 80 | 1.00 | |
| *± *10.00 mg*/*mL Disodium EDTA* (#08) *or ± 4.36 mg*/*mL L-Arginine free base (#09)* | | |

### Example 4: Adalimumab pharmaceutical formulation comprising Histidine-Acetate buffer

Adalimumab was purified according techniques well known in the art. In this Example, the purified Adalimumab was formulated in the presence of Histidine-Acetate buffer along with mannitol, sodium chloride and Polysorbate 80 with or without (#10) one of the additional stabilizers EDTA (#11) and L-Arginine (#12) at concentrations as shown in Table 9. pH of the formulation was adjusted to around pH 5.2. Excipients were added to the protein solution from respective stock solutions to adjust the final concentration and the volume was made up to the desired level with sterile water or Water for Injection. The formulated bulk was distributed in a suitable container (like vials, syringes etc.) for storage under normal and stress conditions. Stability with regard to aggregate formation and deamidation/formation of acidic impurities (species) of the respective formulations was inspected at different time points by HP-Size exclusion chromatography and upon heat exposure by HP-SCX, respectively. Results are shown in Figures 1 to 4.

**Table 9: #10 Histidine-Acetate buffer*.**

| **Components** | **Concentration** | |
|---|---|---|
| | **mg/mL** | **mmol/L** |
| Adalimumab | 50.00 | |
| Sodium chloride | 6.16 | 105.45 |
| L-Histidine base | 0.02 | 1.11 |
| L-Histidine HCl | 0.24 | 11.20 |
| Sodium acetate trihydrate (high grade) | 0.28 | 20.76 |
| Acetic acid (100%) (IN) | 0.03 | 5.52 |
| Mannitol | 12.00 | 65.87 |
| Polysorbate 80 | 1.00 | |
| ***± *10.00 mg*/*mL Disodium EDTA (*#11*) or* ± *4.36 mg*/*mL L-Arginine free base (#12)* | | |

### Example 5: Aggregate content of alternative Adalimumab formulations relative to the reference formulation

Stability of the tested Adalimumab formulations with respect to aggregate formation was measured by HP-SEC during storage at 5°C (Tables 10 and 11) and 25°C (Tables 12 and 13) over a period of 6 months. The values represent the differences in area percentages of aggregated peaks [Δ area%] of the tested Adalimumab formulations compared to the aggregated peaks measured in the respective citrate-phosphate buffered Adalimumab reference formulations. Concerning the L-Arginine containing formulations, only 3-months stability data are available. After 6 months storage at 25°C the Histidine-Citrate and Histidine-Acetate buffered Adalimumab pharmaceutical formulations have apparently a lower aggregation rate compared to the Citrate-Phosphate based reference formulations. Adalimumab formulations based on Citrate buffer alone seem to be more prone to aggregation. Neither the addition of EDTA nor of L-Arginine (L-Arg as determined after 3 months) has a preventive or stabilizing effect on aggregate formation in the alternative formulations. In conclusion the Histidine-Acetate buffered, followed by the Histidine-Citrate buffered, Adalimumab pharmaceutical formulation of the present invention is a more stable alternative to the Citrate-Phosphate buffered Adalimumab formulation on the market.

**Table 10: Aggregate content of alternative Adalimumab formulations in absolute numbers at 5°C.**

| **Formulation** | | | **Aggregates [Area%] at 5°C** | | | | |
|---|---|---|---|---|---|---|---|
| **ID** | **Buffer** | **Stabilizer** | **Initial** | **1 month** | **2 months** | **3 months** | **6 months** |
| 01 | Citrate-Phosphate | - | 4,10 | 3,89 | 3,94 | 3,90 | 4,76 |
| 04 | Citrate | - | 3,65 | 3,95 | 3,78 | 3,98 | 4,68 |
| 07 | Histidine-Citrate | - | 3,63 | 3,68 | 3,49 | 3,68 | 4,35 |
| 10 | Histidine-Acetate | - | 4,02 | 3,67 | 3,64 | 3,65 | 4,28 |
| 02 | Citrate-Phosphate | EDTA | 4,10 | 3,80 | 3,81 | 3,78 | 4,56 |
| 05 | Citrate | EDTA | 3,82 | 3,82 | 3,83 | 3,87 | 4,62 |
| 08 | Histidine-Citrate | EDTA | 3,77 | 3,63 | 3,60 | 3,72 | 4,29 |
| 11 | Histidine-Acetate | EDTA | 3,94 | 3,66 | 3,66 | 3,62 | 4,30 |
| 03 | Citrate-Phosphate | L-Arg | 3,84 | 3,93 | 3,97 | 4,51 | - |
| 06 | Citrate | L-Arg | 3,90 | 3,94 | 4,02 | 4,54 | - |
| 09 | Histidine-Citrate | L-Arg | 3,67 | 3,86 | 3,78 | 4,37 | - |
| 12 | Histidine-Acetate | L-Arg | 3,65 | 3,75 | 3,83 | 4,33 | - |

**Table 11: Aggregate content of alternative Adalimumab formulations relative to the reference formulation at 5°C.**

| **Formulation** | | | **Aggregates [Δ Area%] at 5°C** | | | | |
|---|---|---|---|---|---|---|---|
| **ID** | **Buffer** | **Stabilizer** | **Initial** | **1 month** | **2 months** | **3 months** | **6 months** |
| 04 | Citrate | - | -0,45 | 0,06 | -0,16 | 0,08 | -0,08 |
| 07 | Histidine-Citrate | - | -0,47 | -0,21 | -0,45 | -0,22 | -0,41 |
| 10 | Histidine-Acetate | - | -0,08 | -0,22 | -0,30 | -0,25 | -0,48 |
| 05 | Citrate | EDTA | -0,28 | 0,02 | 0,02 | 0,09 | 0,06 |
| 08 | Histidine-Citrate | EDTA | -0,33 | -0,17 | -0,21 | -0,06 | -0,27 |
| 11 | Histidine-Acetate | EDTA | -0,16 | -0,14 | -0,15 | -0,16 | -0,26 |
| 06 | Citrate | L-Arg | 0,06 | 0,01 | 0,05 | 0,03 | - |
| 09 | Histidine-Citrate | L-Arg | -0,17 | -0,07 | -0,19 | -0,14 | - |
| 12 | Histidine-Acetate | L-Arg | -0,19 | -0,18 | -0,14 | -0,18 | - |

**Table 12: Aggregate content of alternative Adalimumab formulations in absolute numbers at 25°C.**

| **Formulation** | | | **Aggregates [Area %] at 25°C** | | | | |
|---|---|---|---|---|---|---|---|
| **ID** | **Buffer** | **Stabilizer** | **Initial** | **1 month** | **2 months** | **3 months** | **6 months** |
| 01 | Citrate-Phosphate | - | 4,10 | 4,15 | 4,37 | 4,34 | 5,87 |
| 04 | Citrate | - | 3,65 | 3,88 | 4,40 | 4,53 | 5,92 |
| 07 | Histidine-Citrate | - | 3,63 | 3,73 | 3,90 | 3,90 | 4,87 |
| 10 | Histidine-Acetate | - | 4,02 | 3,74 | 3,86 | 3,86 | 4,83 |
| 02 | Citrate-Phosphate | EDTA | 4,10 | 3,93 | 4,15 | 4,06 | 5,39 |
| 05 | Citrate | EDTA | 3,82 | 3,95 | 3,95 | 4,10 | 5,57 |
| 08 | Histidine-Citrate | EDTA | 3,77 | 3,73 | 3,81 | 3,71 | 4,74 |
| 11 | Histidine-Acetate | EDTA | 3,94 | 3,71 | 3,79 | 3,64 | 4,75 |
| 03 | Citrate-Phosphate | L-Arg | 3,84 | 4,09 | 4,36 | 5,17 | - |
| 06 | Citrate | L-Arg | 3,90 | 3,99 | 4,27 | 5,53 | - |
| 09 | Histidine-Citrate | L-Arg | 3,67 | 3,82 | 3,93 | 4,50 | - |
| 12 | Histidine-Acetate | L-Arg | 3,65 | 3,87 | 4,08 | 4,51 | - |

**Table 13: Aggregate content of alternative Adalimumab formulations relative to the reference formulation at 25°C.**

| **Formulation** | | | **Aggregates [Δ Area%] at 25°C** | | | | |
|---|---|---|---|---|---|---|---|
| **ID** | **Buffer** | **Stabilizer** | **Initial** | **1 month** | **2 months** | **3 months** | **6 months** |
| 04 | Citrate | - | -0,45 | -0,27 | 0,03 | 0,19 | 0,05 |
| 07 | Histidine-Citrate | - | -0,47 | -0,42 | -0,47 | -0,44 | -1,00 |
| 10 | Histidine-Acetate | - | -0,08 | -0,41 | -0,51 | -0,48 | -1,04 |
| 05 | Citrate | EDTA | -0,28 | 0,02 | -0,20 | 0,04 | 0,18 |
| 08 | Histidine-Citrate | EDTA | -0,33 | -0,20 | -0,34 | -0,35 | -0,65 |
| 11 | Histidine-Acetate | EDTA | -0,16 | -0,22 | -0,36 | -0,42 | -0,64 |
| 06 | Citrate | L-Arg | 0,06 | -0,10 | -0,09 | 0,36 | - |
| 09 | Histidine-Citrate | L-Arg | -0,17 | -0,27 | -0,43 | -0,67 | - |
| 12 | Histidine-Acetate | L-Arg | -0,19 | -0,22 | -0,28 | -0,66 | - |

### Example 6: Aggregate content of alternative Adalimumab formulations under stress conditions (55°C) relative to the reference formulation

Aggregate formation upon heating the different Adalimumab formulation samples to 55°C was assessed using HP-SEC. In Fig. 2A the aggregate content is shown as area percentages (left columns). The right columns (designated with Δ) represent the differences from the respective Citrate-Phosphate buffered reference formulations for the initial results and the difference from the initial results (change due to stress) for the stressed samples, respectively. Among all tested Adalimumab formulations, the Histidine-Acetate buffered formulation without an additional stabilizer showed the highest stability i.e. the lowest aggregation rate at 55°C; see also Fig. 2B, please note that reference line of citrate-phosphate buffered formulation for 55°C is at Δ 0,34, i.e. above ID #10 (Histidine-Acetate) and ID #08 (Histidine-Citrate + EDTA). Addition of EDTA to the Histidine-Acetate buffer combination seemingly enhances aggregate formation in the respective pharmaceutical formulation (#11) while heating to 55°C. The Adalimumab pharmaceutical formulation buffered with Histidine-Citrate and comprising EDTA shows similar stability at 55°C.

### Example 7: Acidic species content of alternative Adalimumab formulations relative to the reference formulations

Stability of the tested Adalimumab formulations with respect to acidic species formation was measured by SCX-HPLC during storage at 5°C (Tables 14 and 15) and 25°C (Tables 16 and 17) over a period of 6 months (Fig. 3). The values represent the differences in area percentages [Δ area%] of acidic impurity peaks (sum of first and second acidic regions) of the tested Adalimumab formulations compared to the acidic impurity peaks measured in the respective citrate-phosphate buffered Adalimumab reference formulations. Concerning the L-Arginine containing formulations, only 3-months stability data are available. All tested alternative Adalimumab pharmaceutical formulations stored at 5°C have apparently slightly lower levels of acidic impurities than the reference formulations comprising citrate-phosphate buffer, however the differences are not significant (Fig. 3A-C). Stability studies at 25°C revealed that Histidine-Acetate and Histidine-Citrate buffered Adalimumab pharmaceutical formulations show significantly lower levels of acidic species upon storage for 6 months compared to the reference formulations based on Citrate-Phosphate buffer (Fig. 3D-F; this is also true for the 3 months-data in the case of L-Arginine containing formulations, respectively). The Adalimumab formulation buffered with Histidine-Acetate without an additional stabilizer (#10) has the highest relative purity (3.1% lower level of acidic impurities compared to the Citrate-Phosphate buffered reference formulation), whereas Adalimumab formulations based on citrate buffer alone showed the highest amount of acidic impurities. Neither the addition of EDTA nor of L-Arginine (L-Arg as determined after 3 months) has a preventive or stabilizing effect on acidic species formation in the alternative Adalimumab formulations.

**Table 14: Acidic impurities (i.e. acidic species content) of alternative Adalimumab formulations in absolute numbers at 5°C.**

| **Formulation** | | | **Acidic Impurities [Area%] at 5°C** | | | | |
|---|---|---|---|---|---|---|---|
| **ID** | **Buffer** | **Stabilizer** | **Initial** | **1 month** | **2 months** | **3 months** | **6 months** |
| 01 | Citrate-Phosphate | - | 32,25 | 32,32 | - | 32,98 | 33,17 |
| 04 | Citrate | - | 32,34 | 32,55 | - | 32,84 | 32,97 |
| 07 | Histidine-Citrate | - | 32,34 | 32,43 | - | 33,32 | 33,03 |
| 10 | Histidine-Acetate | - | 32,26 | 32,35 | - | 32,54 | 32,79 |
| 02 | Citrate-Phosphate | EDTA | 32,30 | 32,39 | - | 32,89 | 33,03 |
| 05 | Citrate | EDTA | 32,38 | 32,38 | - | 33,01 | 32,68 |
| 08 | Histidine-Citrate | EDTA | 32,31 | 32,37 | - | 33,75 | 32,94 |
| 11 | Histidine-Acetate | EDTA | 32,30 | 32,25 | - | 32,55 | 32,80 |
| 03 | Citrate-Phosphate | L-Arg | 32,64 | 32,99 | 32,47 | 33,01 | - |
| 06 | Citrate | L-Arg | 33,04 | 33,03 | 32,55 | 32,92 | - |
| 09 | Histidine-Citrate | L-Arg | 32,96 | 32,99 | 32,36 | 32,86 | - |
| 12 | Histidine-Acetate | L-Arg | 32,87 | 32,90 | 32,30 | 32,77 | - |

**Table 15: Acidic impurities (i.e. acidic species content) of alternative Adalimumab formulations relative to the reference formulation at 5°C.**

| **Formulation** | | | **Acidic Impurities [Δ Area%] at 5°C** | | | | |
|---|---|---|---|---|---|---|---|
| **ID** | **Buffer** | **Stabilizer** | **Initial** | **1 month** | **2 months** | **3 months** | **6 months** |
| 04 | Citrate | - | 0,09 | 0,23 | - | -0,14 | -0,20 |
| 07 | Histidine-Citrate | - | 0,09 | 0,11 | - | 0,34 | -0,14 |
| 10 | Histidine-Acetate | - | 0,01 | 0,03 | - | -0,44 | -0,38 |
| 05 | Citrate | EDTA | 0,08 | -0,01 | - | 0,12 | -0,35 |
| 08 | Histidine-Citrate | EDTA | 0,01 | -0,02 | - | 0,86 | -0,09 |
| 11 | Histidine-Acetate | EDTA | 0,00 | -0,14 | - | -0,34 | -0,23 |
| 06 | Citrate | L-Arg | 0,40 | 0,04 | 0,08 | -0,09 | - |
| 09 | Histidine-Citrate | L-Arg | 0,32 | 0,00 | -0,11 | -0,15 | - |
| 12 | Histidine-Acetate | L-Arg | 0,23 | -0,09 | -0,17 | -0,24 | - |

**Table 16: Acidic impurities (i.e. acidic species content) of alternative Adalimumab formulations in absolute numbers at 25°C.**

| **Formulation** | | | **Acidic Impurities [Area%] at 25°C** | | | | |
|---|---|---|---|---|---|---|---|
| **ID** | **Buffer** | **Stabilizer** | **Initial** | **1 month** | **2 months** | **3 months** | **6 months** |
| 01 | Citrate-Phosphate | - | 32,25 | 33,92 | - | 38,29 | 44,82 |
| 04 | Citrate | - | 32,34 | 33,84 | - | 38,02 | 46,68 |
| 07 | Histidine-Citrate | - | 32,34 | 33,55 | - | 36,93 | 42,84 |
| 10 | Histidine-Acetate | - | 32,26 | 33,44 | - | 36,69 | 41,71 |
| 02 | Citrate-Phosphate | EDTA | 32,3 | 33,59 | - | 37,96 | 44,15 |
| 05 | Citrate | EDTA | 32,38 | 33,79 | - | 38,22 | 45,68 |
| 08 | Histidine-Citrate | EDTA | 32,31 | 33,44 | - | 36,97 | 42,92 |
| 11 | Histidine-Acetate | EDTA | 32,3 | 33,18 | - | 36,94 | 41,43 |
| 03 | Citrate-Phosphate | L-Arg | 32,64 | 34,76 | 36,00 | 38,21 | - |
| 06 | Citrate | L-Arg | 33,04 | 35,02 | 36,85 | 39,40 | - |
| 09 | Histidine-Citrate | L-Arg | 32,96 | 34,57 | 35,48 | 37,80 | - |
| 12 | Histidine-Acetate | L-Arg | 32,87 | 34,33 | 35,14 | 37,28 | - |

**Table 16: Acidic impurities (i.e. acidic species content) of alternative Adalimumab formulations relative to the reference formulation at 25°C.**

| **Formulation** | | | **Acidic Impurities [Δ Area%] at 25°C** | | | | |
|---|---|---|---|---|---|---|---|
| **ID** | **Buffer** | **Stabilizer** | **Initial** | **1 month** | **2 months** | **3 months** | **6 months** |
| 04 | Citrate | - | 0,09 | -0,08 | - | -0,27 | 1,86 |
| 07 | Histidine-Citrate | - | 0,09 | -0,37 | - | -1,36 | -1,98 |
| 10 | Histidine-Acetate | - | 0,01 | -0,48 | - | -1,60 | -3,11 |
| 05 | Citrate | EDTA | 0,08 | 0,20 | - | 0,26 | 1,53 |
| 08 | Histidine-Citrate | EDTA | 0,01 | -0,15 | - | -0,99 | -1,23 |
| 11 | Histidine-Acetate | EDTA | 0,00 | -0,41 | - | -1,02 | -2,72 |
| 06 | Citrate | L-Arg | 0,40 | 0,26 | 0,85 | 1,19 | - |
| 09 | Histidine-Citrate | L-Arg | 0,32 | -0,19 | -0,52 | -0,41 | - |
| 12 | Histidine-Acetate | L-Arg | 0,23 | -0,43 | -0,86 | -0,93 | - |

### Example 8: Acidic species content of alternative Adalimumab formulations under stress conditions (55°C) relative to the reference formulations

Acidic impurity formation upon heating the different Adalimumab formulation samples to 55°C was assessed using HP-SCX (Fig. 4A). The level of acidic species formation is shown as area percentages (left columns). The right columns (designated with Δ) represent the differences from the respective Citrate-Phosphate buffered reference formulations for the initial results and the difference from the initial results (change due to stress) for the stressed samples, respectively. Among all tested Adalimumab formulations, the Histidine-Acetate buffered formulation without an additional stabilizer (#10) showed the highest stability *i.e.* the lowest acidic impurity formation rate at 55°C. Addition of EDTA to this buffer combination seemingly slightly enhances acidic impurity formation in the respective formulation while heating to 55°C.

### Example 9: Biological activity of alternative Adalimumab formulations under stress conditions relative to the reference formulation

### Principle of method

The biological activity of the test sample is determined over time under different stress conditions in comparison with the biological activity of HUMIRA® reference solution. The effect of inhibiting cell death by the reference solution and the test sample is compared on L929 cells treated with TNFα and Actinomycin-D, which is detected with AlamarBlue® reagent. During the treatment a medium containing L929 cells in the same concentration is added into the appropriate wells of a 96-well microtiter plate, and incubated for 48 hours. After the incubation 9-point dilution series are prepared in three replicates from both the reference solution and the test sample, in the same concentration on one dilution plate, then the solution of TNFα is added, and the mixtures are incubated for 15 minutes at room temperature. During the incubation period the solution of Actinomycin-D is added into the wells of the microtiter plate which contain the cells, then the solution of TNFα and the reference solution, or the test sample is added to the cells. After 24 hours the AlamarBlue® reagent is added to the cells, then after another 24-hour incubation the fluorescent signals are detected, which are proportional to the number of viable cells. The evaluation of the biological activity of the test sample relative to the reference solution is based on the comparison of the 5-parameter sigmoid curves obtained from points of the dilution series. After analysing the characteristics of the fit (regression, linearity, and parallelism), relative biological activity of the sample is calculated from the distances of the curves; the optimum value is 100 %.

### Necessary materials and equipment

### Materials

- Actinomycin-D (Sigma A9415)
- AlamarBlue® (Invitrogen DAL 1025)
- DMEM, high glucose, GlutaMAX, HEPES (Gibco 32430)
- Ethanol (Merck 1.00983.1000)
- FBS, Foetal Bovine Serum (Heat-Inactivated) (Gibco 10500-056)
- HUMIRA® reference stock solution (Abbott, 50 mg/mL)
- Kanamycin sulphate, Streptomyces kanamyceticus, Cell Culture-Tested (Calbiochem, 420411)
- PBS pH 7.4
- Recombinant Human TNFα (R&D Systems, 210TA/CF)
- TrypLETM Express (1x), phenol red (Gibco, 12605-010)
- Trypan Blue solution (Sigma T8154)
- Stock solution of the test sample
- Water, purified, HPLC grade

### Equipment

- Tissue culture flask (TPP 711090076)
- 96-well microtiter plate (BD Falcon 3072)
- 50-mL centrifuge tube (BD Falcon 352070)
- Eppendorf Research® pipettes
- Cat. No.: 3111 000.130 (volume range: 2-20 µL, inaccuracy: ±1.2%, imprecision: ≤0.6%)
- Cat. No.:3111 000.157 (volume range: 20-200 µL, inaccuracy: ±1.0%, imprecision: ≤0.3%)
- Cat. No.:3111 000.165 (volume range: 100-1000 µL, inaccuracy: ±0.6%, imprecision: ≤0.2%)
- Eppendorf multichannel pipette
- Cat. No.: 3114 000.131 (volume range: 10-100 µL, inaccuracy: ±0.8%, imprecision: ≤0.3%)
- Eppendorf tubes (Eppendorf 0030 108.116 Protein LoBind Tubes, 1.5 mL, PCR clean)
- Millex-GV filter unit, 0.22 µm, PVDF, 33 mm, (Millipore SLGV033RB)
- Biosafety II laminar box
- CO2 incubator
- Biotek Synergy 2 plate reader, Gen5 Secure software
- Beckman Coulter Allegra X-22R table centrifuge with interchangeable rotor
- Sigma Pasteur pipette (Sigma-Aldrich, S6018-250EA)
- Serological pipette, 10 mL (TPP 94010)
- Serological pipette, 25 mL (TPP 94024)
- Bürker chamber (0.0025 mm2, Hirscmann)
- Nucleocounter automatic cell-counter
- Agilent Bravo liquid handling robot
- Water purifier (Sartorius Arium 611VF)
- Analytical balance (Mettler Toledo AX205)
- Magnetic stirrer (IKA Ret basic)
- Materials and equipment of the same quality can be used as well.

### Preparation of solutions

### Complete medium

- 90 mL DMEM medium
- 10 mL FBS
- 200 µL Kanamycin solution (50 mg/mL, aqueous solution of purified water)
The medium can be used for no longer than one week after preparation, the components are not sterilised by the supplier; the medium has to be sterilised by filtration.
- 10x PBS
- 40 g NaCl
- 5.8 g Na2HPO4
- 1 g KH2PO4
- 1 g KCl
solved in 500 mL purified water, pH 7.0
(It is not necessary to set the pH value of the 1x PBS, it will be at about pH 7.4-7.5.)

### Preparation of TNFα stock solution

Prepare a stock solution of 1 mg/mL concentration from the TNFα. Add 50 µL sterile filtered PBS to 50 µg lyofilized TNFα stored at -20°C, and then solve by gentle shaking.
The TNFα stock solution stored at -70°C can be used for 3 months after preparation.

### Preparation of Actinomycin-D stock solution

Prepare a stock solution of 1 mg/mL concentration from Actinomycin-D. Add 2 mL ethanol to 2 mg lyofilized Actinomycin-D, and then solve with a vortex.
The Actinomycin-D stock solution stored at -20°C can be used for 3 months after preparation.

### Dilution of TNFα

Prepare a solution of 112 pg/mL concentration from the 1 mg/mL TNFα stock solution stored at -70°C in four dilution steps, which is further diluted 4-fold in the wells, and the final concentration is 28 pg/mL.

### Dilution steps:

1. 100x (198 µL complete medium + 2 µL TNFα stock solution)
2. 100x (990 µL complete medium + 10 µL from the previous dilution step)
3. 10x (900 µL complete medium + 100 µL from the previous dilution step)
4. 89x (on 3 plates: 19.8 mL complete medium + 225 µL from the previous dilution step)

### Dilution of Actinomycin-D

Prepare a solution of 4 µg/mL concentration from the 1 mg/mL Actinomycin-D stock solution stored at -20°C by 250x dilution (20 µL stock solution + 5 mL complete medium on each plate), which is further diluted 4-fold in the wells, and the final concentration is 1 µg/mL.

### Preparation of reference solution and test solution

Dilute the HUMIRA® reference stock solution (50 mg/mL) to 7.2 µg/mL in three steps. Starting from the minimum of 10 µL stock solution the subsequent dilution steps are: 10 x, 100x, and 6.94x. Transfer 50 µL from the solution diluted to the concentration of 7.2 µg/mL to appropriate wells of the dilution plate, where the final concentration of reference solution becomes 2.4 µg/mL.

### Dilution steps:

1. 10x (90 µL complete medium + 10 µL HUMIRA® reference stock solution or test solution)
2. 100x (990 µL complete medium + 10 µL from the previous dilution step)
3. 6.94x (178.2 µL complete medium + 30 µL from the previous dilution step)

The nominal concentration of the HUMIRA® reference stock solution is 50 mg/mL; from this prepare a solution of 7.2 µg/mL concentration in a way that the difference between the actual concentration and the nominal concentration is corrected in the last dilution step. Thus, measure the quantity from the solution obtained in the second dilution step so that the concentration of the third solution is 7.2 µg/mL. (Initial concentration (µg/mL) / 1000 = concentration after the first two dilution steps (µg/mL); Degree of third dilution step = concentration after the first two dilution steps (µg/mL) / 7.2 (µg/mL); Weighing: total volume of 3rd solution (µL) / degree of 3rd dilution step = x (µL) from the 2nd dilution) Dilute the stock solution of the test sample to 7.2 µg/mL similarly to the reference solution.

### Performance of test, test conditions

Perform every step under sterile condition.

### Plating of cells - Day 1

### Preparation of cell suspension

Three days prior to plating the cells, transfer about 2.5 x 10⁶ cells into 25 mL of complete medium in a tissue culture flask. On the day of plating the cells, after withdrawing the medium loosen the cells attached to the surface with 3 mL of TrypLETM Express solution, and then suspend in 7 mL of complete medium. Using the Bürker chamber with trypan blue staining (BTCH-BIOASSAY-BÜRKER-01), or with Nucleocounter (BTCH-BIOASSAY-SEJTSZ-01), determine the cell number and the viability. If the portion of viable cells is less than 90%, do not use the cell culture. Set the cell number to 1.6x 10⁵ cell/mL in the medium necessary for the assay. 6 mL of cell suspension is necessary on one plate, 50 µL per each cell. Use the cell suspension within 1 hour. Incubate the plate for 48 hours at 37°C in 5% CO₂ 85% RH atmosphere.

### Treatment of cells - Day 3

**Table 17 Composition of solutions and their arrangement on the plate.**

| | **1** | **2** | **3** | **4** | **5** | **6** | **7** | **8** | **9** | **10** | **11** | **12** |
|---|---|---|---|---|---|---|---|---|---|---|---|---|
| **A** | Medium | Medium | Medium | Medium | Medium | Medium | Medium | Medium | Medium | Medium | Medium | Medium |
| **B** | Medium | Reference 1 | Reference | Reference | Reference | Reference | Reference | Reference | Reference | Reference | Cell ctrl | Medium |
| **C** | Medium | Sample 1 | Sample | Sample | Sample | Sample | Sample | Sample | Sample | Sample | Cell ctrl | Medium |
| **D** | Medium | Reference | Reference | Reference | Reference | Reference | Reference | Reference | Reference | Reference | ActD ctrl | Medium |
| **E** | Medium | Sample 2 | Sample | Sample | Sample | Sample | Sample | Sample | Sample | Sample | ActD ctrl | Medium |
| **F** | Medium | Reference 3 | Reference | Reference | Reference | Reference | Reference | Reference | Reference | Reference | TNFα ctrl | Medium |
| **G** | Medium | Sample 3 | Sample | Sample | Sample | Sample | Sample | Sample | Sample | Sample | TNFα ctrl | Medium |
| **H** | Medium | Medium | Medium | Medium | Medium | Medium | Medium | Medium | Medium | Medium | Medium | Medium |

Medium: complete medium 200 µL/well (wells: A 1-12, H 1-12, B-G 1, B-G 12);
Cell ctrl: cell suspension 50 µL/well + complete medium 150 µL/well (11 B-C);
ActD ctrl: cell suspension 50 µL/well + Actinomycin-D (4 µg/mL) 50 µL/well + complete medium 100 µL/well (11 D-E);
TNFα ctrl: cell suspension 50 µL/well + TNFα solution (112 µg/mL) 100 µL/well + Actinomycin-D (4µg/mL) 50 µL/well (11 F-G);
Reference: solutions of the dilution series prepared from the reference solution 50 µL/well + cell suspension 50 µL/well + TNFα solution (112 µg/mL) 50 µL/well + Actinomycin-D (4 µg/mL) 50 µL/well, 1-3: dilution series prepared from independent dilution series;
Sample: solutions of the dilution series prepared from the test solution 50 µL/well + cell suspension 50 µL/well + TNFα solution (112 µg/mL) 50 µL/well + Actinomycin-D (4 µg/mL) 50 µL/well, 1-3: dilution series prepared from independent dilution series.

### Treatment procedure

Prepare independent dilution series from the HUMIRA® reference solution and the test solution, three replicates from each solution. On the microtiter plate, measure 100 µL of complete medium from the 2nd through the 9th columns, and then add 50 µL from the previously diluted 7.2 µg/mL solutions into the 2nd column alternately, so in wells B, D and F there will be HUMIRA® reference solution of 2.4 µg/mL concentration, and in wells C, E and G there will be test solution of 2.4 µg/mL concentration. Transfer the concentrated solutions in pairs into the wells, one reference solution and then one test solution, etc. Starting from these solutions, prepare 9-point dilution series for the preparation of three-fold dilutions. For this purpose transfer 50 µL of solution from the next, one degree more concentrated dilution point, then suspend, and discard the excess 50 µL from the last well. Transfer 100 µL of TNFα solution (112 pg/mL) to each 100µL point in the prepared dilution series, and then incubate for 15 minutes at room temperature. During the incubation period, transfer 50 µL of Actinomycin-D solution into the wells of the microtiter plate containing the cells, then transfer the control solution into the appropriate wells as well. After the incubation time, pipette 100 µL from each point of the TNFα dilution series into the appropriate wells of the microtiter plate containing the cells, and then incubate the plate for 24 hours at 37°C in 5% CO2, 85% RH atmosphere.

### Staining - Day 4

Transfer 25 µL of AlamarBlue® reagent into every chamber of the plate, and then incubate the plate for 24 hours at 37°C in 5% CO2, 85% RH atmosphere.

### Test - Day 5

Detect the fluorescence intensity of samples on the plate with multimode plate reader set in fluorescence mode, at 530 nm extinction and 590 nm emission wavelength values (sensitivity: 45 nm).

### Evaluation

Perform the evaluation by using PLA 2.0 (Stegman System, Germany) software, based on the "5 parameter logistic curve (full curve)" model. Use the following setting in the software:
- Analysis: 5 parameter logistic curve (full curve)
- Hypothesis testing: ANOVA based on pure error separation
- Parallelism: (95% confidence)
- Configuration: Linear detection type/ Full range
On the basis of fluorescence values measured by the software, determine the concentration-effect curves characteristic for the test solution and the reference solution, and fit 5-parameter logistic equation on these curves by applying the given conditions. The program calculates the relative biological activity of the test solution related to the reference solution (HUMIRA®) based on the distance of the linear part of fitted curves on the x-axis.

In addition, the software determines the following system suitability criteria based on F-test:
For parallel measurements: CV% < 15%
In the case CV > 15%, if it is proven by the outlier test according to the Pharmacopoeia, that one point from the parallels is an outlier, it can be withdrawn manually, and then the plate is reevaluated.
Regression: Fregression > Fcritical (95%)
Linearity: Fnon-linear < Fcritical (95%)
Parallelism: Fnon-parallel < Fcritical (95%)
Relative confidence interval: between 74% and 136%

### Other system suitability criteria:

- Mean of cell control values / Mean of ActD control values should be between 1-1.5;
- Mean of TNFα control values in every case should be less than the mean value corresponding to the lowest treatment concentration of the reference solution;
- Mean of cell control values in every case should be more than the mean value corresponding to the highest treatment concentration of the reference solution.

Determination of the biological activity of the test samples over time under different stress conditions in comparison with the biological activity of HUMIRA® reference solution showed that each Adalimumab formulation of the present invention is superior in relation over the time (Fig. 5B) or over the HUMIRA® reference solution (Fig. 5C) in at least one of the storage and stress conditions tested, *i.e.* after 3 months at 5 °C and 25 °C, after 6 months at 5 °C and 25 °C, after a freeze-thaw cycle, after mechanical stress as well as after heat stress at 55°C (Fig. 5A-C). For example, the Histidine-Acetate buffered formulation without an additional stabilizer (sample ID NO. #10) showed the highest ability to neutralize TNFα after 6 months at 5 °C and 25 °C, followed by Histidine-Acetate buffered formulation with L-Arg (sample ID NO. #12) relative to time point 0. Further, the Histidine-Acetate buffered formulation with L-Arg (sample ID NO. #12) showed the best performance after 3 months at 5 °C and 25 °C, after a freeze-thaw cycle as well as mechanical stress relative to time point 0. The Histidine-Citrate buffered formulation without an additional stabilizer (sample ID NO: #07) showed the highest TNFα neutralization after 6 months at 5 °C as well as after heat stress at 55°C relative to time point 0. All remaining Adalimumab formulations show similar rates of degradation to the investigated stress factor (Fig. 5A, B). Relative to the reference formulation, the Histidine-Acetate buffered formulation without an additional stabilizer (sample ID NO. #10) showed the highest ability to neutralize TNFα after 6 months at 5 °C and 25 °C, followed by Histidine-Citrate (sample ID NO. #07) after 6 months at 5 °C. The Histidine-Acetate buffer formulation with L-Arg (sample ID NO. #12) showed overall the best performance to all investigated stress factors (Fig. 5A, C). The experiments summarized in Example 9 and Fig. 5 were performed with a second batch of alternative Adalimumab formulations. TNFα neutralization percentages of alternative Adalimumab formulation of the present invention are expressed relative to the TNFα neutralization property of the HUMIRA® reference solution, *i.e.* values > 100 % indicate that the relative TNFα neutralizing activity retained by the Adalimumab formulation of the present invention is higher that retained by the reference solution under the given condition.

In summary, each Adalimumab formulation of the present invention represents with respect to its biological activity an improved storage-stable liquid pharmaceutical antibody formulation.

### References

Arakawa T, Philo JS, Ejima D, Tsumoto K, and Arisaka F (2006). Aggregation analysis of therapeutic proteins, part 1. Bioprocess International 4 (10), 42-49.
Chen B, Bautista R, Yu K, Zapata GA, Chamow SM, et al. (2003). Influence of histidine on the stability and physical properties of a fully human antibody in aqueous and solid forms. Pharm Res 20:1952-1960.
Paborji M, Pochopin NL, Coppola WP, Bogardus JB (1994). Chemical and physical stability of chimeric L6, a mouse-human monoclonal antibody. Pharm Res 11:764-771.
Sarno MEC, Vasquez RA, Yung S-G, Graf CR (1999). Stable intravenously-administrable immune globulin preparation. Baxter International Inc, US patent US5945098.
EMA/CHMP/BWP/247713/2012 (22.05.2014). Guideline on similar biological medicinal products containing biotechnology-derived proteins as active substance: quality issues (revision 1)
U.S. Department of Health and Human Services, Food and Drug Administration (February 2012). Guidance for Industry - Scientific Considerations in Demonstrating Biosimilarity to a Reference Product" (DRAFT GUIDANCE).
Public Health Service (PHS) Act §262. Public Health Service Act, Title 42: The Public Health And Welfare, Chapter 6a: Public Health Service, Subchapter II: General Powers and Duties, Part F: Licensing of Biological Products and Clinical Laboratories, Subpart 1 - Biological Products: §262 Regulation of Biological Products (http://www.fda.gov/regulatoryinformation/legislation/ucml48717.htm).
Kappelgaard AM, Bojesen A, Skydsgaard K, Sjogren I, Laursen T (2004). Liquid growth hormone: preservatives and buffers. Horm Res.62 Suppl 3:98-103.
Frenken LA1, van Lier HJ, Jordans JG, Leunissen KM, van Leusen R, Verstappen VM, Koene RA (1993). Identification of the component part in an epoetin alfa preparation that causes pain after subcutaneous injection.Am J Kidney Dis. 22(4):553-6.
Fransson J, Espander-Jansson A. Local tolerance of subcutaneous injections (1996). J Pharm Pharmacol. 48(10):1012-5.
Kolhe P, Amend E, Singh SK (2010). Impact of freezing on pH of buffered solutions and consequences for monoclonal antibody aggregation. Biotechnol Prog. 26(3):727-33.
Gatlin LA and Gatlin CAB (1999). Formulation and administration techniques to minimize injection pain and tissue damage associated with parenteral products. In Injectable Drug Development: Techniques to Reduce Pain and Irritation (Gapta PK and Brazeau GA eds) Interpharm Press, Denver 401-425.
Roskos LK, Davis CG and Schwab GM (2004). The clinical pharmacology of therapeutic monoclonal antibodies. Drug Devel Res 62(3): 108-120.
Pennica D, Nedwin GE, Hayflick JS, Seeburg PH, Derynck R, Palladino MA, Kohr WJ, Aggarwal BB, Goeddel DV (1984). Human tumour necrosis factor: precursor structure, expression and homology to lymphotoxin. Nature 312 (5996):724-729
Wang W (1999). Instability, stabilization, and formulation of liquid protein pharmaceuticals. Int J Pharm 185:129-188.
Chi EY, Krishnan S, Randolph TW and Carpenter JF (2003). Physical stability of proteins in aqueous solution: mechanism and driving forces in nonnative protein aggregation. Pharmaceutical Research 20: 1325-1336.
Vermeer AWP and Norde W (2000). The thermal stability of immunoglobulin: Unfolding and aggregation of a multi-domain protein. Biophys J 76: 394-404.
Moore JM, Patapoff TW and Cromwell ME (1999). Kinetics and thermodynamics of dimer formation and dissociation for a recombinant humanized monoclonal antibody to vascular endothelial growth factor. Biochemistry 38 (42): 13960-13967.
Szenczi A, Kardos J, Medgyesi G and Zavodszky N (2006). The effect of solvent environment on the conformation and stability of human polyclonal IgG in solution. Biologicals 34(1): 5-14.
Wan HZ, Kaneshiro S, Frenz J and Cacia J (2001). Rapid method for monitoring galactosylation levels during recombinant antibody production by electrospray mass spectrometry with selective-ion monitoring. J Chrom A 913(1-2):437-446.
Zheng JY, Janis LJ (2005). Influence of pH, buffer species, and storage temperature on physicochemical stability of a humanized monoclonal antibody LA298. Int J Pharm 308:46-51.
Son K, Kwon C.Stabilization of human epidermal growth factor (hEGF) in aqueous formulation (1995). Pharm Res.12(3):451-4.
Bam NB, Randolph TW, Cleland JL (1995). Stability of protein formulations: investigation of surfactant effects by a novel EPR spectroscopic technique. Pharm Res. 12(1):2-11.
Wade AM and Tucker HN (1998). Antioxidant characteristics of L-histidine. J Nutr Biochem 9 (6): 308-315.
International Conference on Harmonization of Technical Requirements of Pharmaceuticals for Human Use. Quality of Biotechnological Products. Stability Testing of Biotechnological/Biological Products (ICH Q5C).
Cleland JL, Powell MF and Shire SJ (1993). The development of stable protein formulations: A close look at protein aggregation, deamidation, and oxidation. Crit Rev Ther Drug Carrier Syst 10(4): 307-377.
Carpenter JF, Kendrick BS, Chang BS, Manning MC and Randolph TW (1999). Inhibition of stress-induced aggregation of protein therapeutics. Meth Enzymol 309: 236-255.
Remmele RL Jr, Callahan WJ, Krishnan S, Zhou L, et al (2006). Active dimer of Epratuzumab provides insight into the complex nature of an antibody aggregate. J Pharm Sci 95(1): 126-145.
Chelius D, Rehder DS, Bondarenko PV (2005). Identification and characterization of deamidation sites in the conserved regions of human immunoglobulin gamma antibodies. Anal Chem. 77(18):6004-11.

## Claims

1. A method of obtaining a long-term stable phosphate-buffer free pharmaceutical formulation of an anti-TNFa antibody, preferably Adalimumab comprising formulating the antibody in an L-Histidine-citrate or L-Histidine-acetate buffer.

2. The method of claim 1, wherein the pH of the formation is 5 to 5.5.

3. The method of claim 1 or 2, wherein the concentration of the antibody is between about 30-100 mg/mL, preferably between 45 to 55 mg/mL.

4. The method of any one claims 1 to 3, wherein the pharmaceutical formulation further comprises one or more excipients, preferably selected from polyols, salts, amino acids, surfactants and water as solvent.

5. The method of any one claims 1 to 4, wherein the pharmaceutical formulation is designed for parenteral administration, preferably for intravenous injection, or for intramuscular injection, more preferably for subcutaneous administration.

6. The method of any one claims 1 to 5, wherein the pharmaceutical formulation is provided in a container or set of containers for use as a medicament for use in the treatment of a disorder in which TNFα activity is detrimental, preferably wherein the disorder in which TNFα activity is detrimental is selected from the group consisting of an autoimmune or inflammatory disorder.

7. The method of claim 6, wherein the container is a prefilled syringe, injection pen, ampoule, bottle, autoinjector or an infusion bag, preferably being closed under nitrogen protective atmosphere.

8. The method of any one claims 1 to 7, wherein the antibody is Adalimumab and the dose strength of Adalimumab as provided within the pharmaceutical formulation is 20, 40, 80, 120 or 160 mg, preferably wherein Adalimumab is designed to be administered at a total dose of 20, 40 or 80 mg.

9. The method of any one of claims 1 to 8, wherein the pharmaceutical formulation is **characterized by**:
(a) having a content of aggregated species of Adalimumab after storage at 5°C for 3 to 6 months of less than 5 %;
(b) having a content of aggregated species of Adalimumab after storage at 25°C for 3 to 6 months of less than 5 %;
(c) having a content of acidic species of Adalimumab after storage at 25°C for 3 to 6 months of less than 40 %;
(d) having a content of aggregated species of Adalimumab under heat stress conditions for 10 minutes at 55°C of less than 5 %;
(e) having a content of acidic species of Adalimumab under heat stress conditions for 10 minutes at 55°C of less than 40%; and/or
(f) retaining TNFα neutralizing activity of at least 80% after storage of 6 months at a temperature of 5°C or 25°C, and/or after being subjected to heat stress for 10 minutes at 55°C, freeze-thaw conditions at -20°C to 5°C and/or mechanical stress;
wherein the aggregated species are measured by Size Exclusion High Performance Liquid Chromatography (HP-SEC) and the acidic species are measured by Strong Cation Exchange (SCX) High Performance Liquid Chromatography (SCX-HPLC).

10. A long-term stable phosphate-buffer free pharmaceutical formulation of an anti-TNFa antibody, preferably Adalimumab obtainable by a method of any one of claims 1 to 9.

11. Use of an L-Histidine-citrate or L-Histidine-acetate buffer in the formulation of a long-term stable phosphate-buffer free pharmaceutical formulation of an anti-TNFa antibody, preferably Adalimumab.

12. The use of claim 11, wherein the pharmaceutical formulation shows increased long-term stability in terms of aggregate formation and generation of acidic species, preferably as defined in claim 9.

13. The use of claim 11 or 12, wherein the pH of the formation is 5 to 5.5 and the concentration of the antibody is between 45 to 55 mg/mL.

14. The use of any one claims 11 to 13, wherein the pharmaceutical formulation further comprises one or more excipients, preferably wherein the excipient is selected from polyols, salts, amino acids, surfactants and water as solvent.

15. The use of any one claims 11 to 14, wherein the pharmaceutical formulation is designed for parenteral administration, preferably for intravenous injection, or for intramuscular injection, more preferably for subcutaneous administration for use in the treatment of a disease selected from the group consisting of autoimmune disorders and malignant diseases.
